(19) Europäisches Patentamt
European Patent Office
Office européen des brevets

(11) EP 2 168 992 A1

(12) EUROPEAN PATENT APPLICATION
published in accordance with Art. 153(4) EPC

(43) Date of publication:
**31.03.2010 Bulletin 2010/13**

(21) Application number: **08791053.5**

(22) Date of filing: **10.07.2008**

(51) Int Cl.:
***C08F 230/02*** *(2006.01)* ***A61K 31/785*** *(2006.01)*
***A61P 3/12*** *(2006.01)*

(86) International application number:
**PCT/JP2008/062505**

(87) International publication number:
**WO 2009/008480 (15.01.2009 Gazette 2009/03)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **11.07.2007 JP 2007182127**
**04.12.2007 JP 2007313554**

(71) Applicant: **Toray Industries, Inc.**
**Tokyo 103-8666 (JP)**

(72) Inventors:
- **INOUE, Atsushi**
  **Kamakura-shi**
  **Kanagawa 248-8555 (JP)**
- **SUYAMA, Kazuharu**
  **Kamakura-shi**
  **Kanagawa 248-8555 (JP)**
- **MINAKAMI, Satoshi**
  **Kamakura-shi**
  **Kanagawa 248-8555 (JP)**
- **MIYAMOTO, Mitsuko**
  **Kanagawa 248-8555 (JP)**
- **ITABA, Shoichi**
  **Kamakura-shi**
  **Kanagawa 248-8555 (JP)**

(74) Representative: **Prüfer & Partner GbR**
**European Patent Attorneys**
**Sohnckestrasse 12**
**81479 München (DE)**

# (54) CROSSLINKED POLYALLYLAMINE OR ACID ADDITION SALT THEREOF, AND USE THEREOF FOR MEDICAL PURPOSES

(57) Disclosed are a cross-linked polyallylamine or an acid addition salt thereof having both high phosphate adsorption capability and low degree of swelling, and a medical use thereof. A cross-linked polyallylamine or an acid addition salt thereof was provided, which is obtained by copolymerization of allylammonium dihydrogen phosphate with an acid addition salt of *N,N'*-diallyl-1,3-diaminopropane in an amount of 5 to 25 mol% with respect to the amount of the allylammonium dihydrogen phosphate, the cross-linked polyallylamine or an acid addition salt thereof having a phosphate adsorption capacity of 2.7 to 5.0 mmol/g; and a degree of swelling of 2.0 to 5.0. This cross-linked polyallylamine or an acid addition salt thereof is useful as a therapeutic or prophylactic agent for hyperphosphatemia.

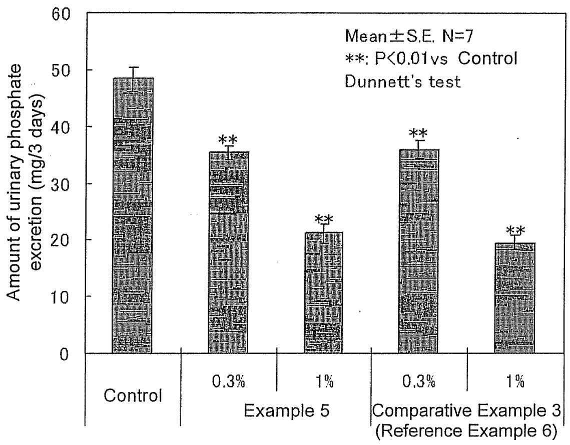


Fig.1

EP 2 168 992 A1

**Description**

TECHNICAL FIELD

The present invention relates to a cross-linked polyallylamine or an acid addition salt thereof, and medical use thereof.

BACKGROUND ART

Patients with renal function impairment often suffer from hyperphosphatemia due to decreased phosphate excretion. Hyperphosphatemia causes serious abnormality in metabolism of calcium and phosphate, leading to decreased serum calcium, promotion of production and secretion of PTH, ectopic calcification, and renal osteodystrophy due to inhibition of vitamin D activation. Even after starting dialysis because of renal failure, the above-described disease state continues unless the homeostasis of phosphate is maintained. Thus, therapy of hyperphosphatemia is indispensable for patients with renal failure who are dialyzed or not dialyzed. At present, therapy of hyperphosphatemia is carried out by diet therapy, or pharmacotherapy by an oral phosphate adsorption agent. However, it is thought that diet therapy is not sufficient for amelioration of hyperphosphatemia and that therapy for these patients requires use of a phosphate adsorption agent.

Examples of conventional phosphate adsorption agents which have been widely used include inorganic salts such as aluminum salts and calcium salts. Ingested aluminum salts and calcium salts bind to phosphate in the intestine to form insoluble phosphates, which inhibit absorption of phosphate. However, administration of aluminum salts causes accumulation of aluminum, leading to brain diseases, osteomalacia and the like, which is problematic. Further, administration of calcium salts causes hypercalcemia, leading to early death of patients by calcification of the aorta and ectopic calcification, which is problematic. Recently, lanthanum carbonate which is one of lanthanum salts has been placed on the market. However, since intestinal absorption and accumulation of lanthanum has been observed, there is a concern about the safety aspect during chronic administration.

Recently, as an oral phosphate adsorption agent, sevelamer hydrochloride, a polyallylamine which is an organic polymer has been placed on the market. Since sevelamer hydrochloride does not cause the side effects observed upon administration of the above-described inorganic salts, it is widely used for therapy of hyperphosphatemia. Sevelamer hydrochloride is a poly(allylamine/epichlorohydrin) and a production method thereof includes the steps of polymerizing allylamine hydrochloride to obtain polyallylamine hydrochloride and subsequent cross-linking by reacting the product with epichlorohydrin in an aqueous sodium hydroxide solution (Patent Literature 1).

However, sevelamer hydrochloride is said to require administration at a high dose in order to remarkably reduce phosphate absorption. Further, since sevelamer hydrochloride absorbs water in the gastrointestinal tract, it causes side effects such as constipation, abdominal pain and abdominal distension, which is problematic. It is also reported that such side effects may lead to serious side effects such as intestinal perforation and intestinal obstruction (Non-patent Literature 1). Further, the expression frequencies of side effects are known to be dose-dependent (Non-patent Literature 2). Since, in sevelamer hydrochloride, there exist these side effects caused by swelling, administration thereof at an amount required for sufficient inhibition of phosphate absorption is often difficult, and it needs to be used in combination with a calcium formulation at present. Therefore, development of an oral phosphate adsorption agent having a lower degree of swelling, which replaces sevelamer hydrochloride is demanded (Non-patent Literature 3).

Further, the ion-exchange rate of an ion-exchange resin such as a polyallylamine decreases and the phosphate adsorption capability thereof decreases in cases where the degree of swelling is low, so that it is considered to be difficult to achieve a low degree of swelling while maintaining a high phosphate adsorption capacity. Further, in addition to phosphate, there exist acids such as bile acids in the intestine, and these are known to compete with each other in terms of adsorption to polyallylamine and the like.

Since sevelamer hydrochloride has a lower selectivity to phosphate than to bile acids, there is a possibility that not only its phosphate adsorption capacity decreases, but also it causes uptake inhibition of fat-soluble vitamins due to adsorption of bile acid in the intestine (Non-patent Literature 1). Therefore, development of an oral phosphate adsorption agent having a higher phosphate selectivity, which replaces sevelamer hydrochloride is demanded (Non-patent Literature 3).

By the way, reported examples of the production method of a cross-linked polyallylamine include, other than a method wherein a polyallylamine is synthesized first and reacted with a compound which is capable of reacting with amino groups of the polyallylamine at multiple sites, as in the method of producing sevelamer hydrochloride, a method wherein an allylamine salt is copolymerized with a polyfunctional monomer (Patent Literature 2).

However, in the method described in Patent Literature 2, the amount of the polyfunctional allylamine derivative to be added is described to be not more than 2 mol% with respect to the allylamine salt, and the cross-linked polyallylamine obtained in the Examples was soluble in water, so that it is not appropriate for use as an oral phosphate adsorption

agent. Further, there is no description on the degree of swelling of the obtained cross-linked polyallylamine, and no attempt to decrease the degree of swelling was made at all. Further, in the Examples, there are only examples using an allylamine hydrochloride, and differences in properties of the polymers depending on the types of the acids used in the polymerization have not been studied. Thus, it is said that the cross-linked polyallylamine described in Patent Literature 2 cannot be used as an oral phosphate adsorption agent having a low degree of swelling.

In Patent Literature 3, there is a report on a production method of a polyallylamine wherein, in the synthesis of its homopolymer, a phosphate of allylamine is used as a monomer for polymerization. However, in this report, there is no description on synthesis of a cross-linked polyallylamine by cross-linking polymerization.

In Patent Literature 4, there is a report on a phosphate adsorption agent produced using the so-called molecular imprinting, wherein the polymerization is carried out in the presence of phosphate to obtain a polymer which was given an affinity to phosphate ions. In this method, a porous phosphate-imprinted polymer was obtained by carrying out polymerization after mixing monomers with potassium dihydrogenphosphate in the presence of a diluent such as 2-propanol. However, in this report, only methods using as the monomers an acrylic acid derivative are disclosed, without disclosing methods using allylamine. Further, the above-described porous phosphate-imprinted polymer is inferior to sevelamer hydrochloride in terms of the phosphate adsorption capacity; its selectivities to other ions such as bile acids are not investigated; and its volume increases about tenfold upon swelling; so that it is said that it cannot be used as an oral phosphate adsorption agent having a low degree of swelling.

In Patent Literature 5, as a method to obtain a polymer having a low degree of swelling, cross-linking of the surfaces of particles of a cross-linked polymer is reported. However, the method described in Patent Literature 5 is a disclosure about a water-absorbing polymer, and not a disclosure about a polymer which exerts the phosphate adsorption capability and, at the same time, has a low degree of swelling.

Patent Literature 1: JP 3113283 B
Patent Literature 2: JP 10-330427 A
Patent Literature 3: JP 2-14364 B
Patent Literature 4: US 2005/0276781 A1
Patent Literature 5: JP 11-302391 A
Non-patent Literature 1: Drug Interview Form: Renagel (registered trademark) Tablet 250 mg, 8th Revised Edition, 2005, p. 21
Non-patent Literature 2: Review Report, Notification No. 3850 of National Institute of Health Sciences (Nov. 28, 2002)
Non-patent Literature 3: Hiroyoshi INOUE, "Phosphate excretion enhancers/absorption inhibitors in the future", Kidney and Dialysis, 2003, Vol. 55, p. 941-944

DISCLOSURE OF THE INVENTION

PROBLEMS TO BE SOLVED BY THE INVENTION

In view of this, the present invention aims to provide a cross-linked polyallylamine or an acid addition salt thereof, which has both a high phosphate adsorption capacity and a low degree of swelling, and a medical use thereof.

MEANS FOR SOLVING THE PROBLEMS

The present inventors intensively studied on an oral phosphate adsorption agent to discover a cross-linked polyallylamine and an acid addition salt thereof having a low degree of swelling and a high phosphate absorption capacity, as a result of copolymerization of allylammonium dihydrogen phosphate with an acid addition salt of *N,N'*-diallyl-1,3-diaminopropane as a cross-linking agent in an amount of 5 to 25 mol% with respect to the amount of the allylammonium dihydrogen phosphate.

That is, the present invention provides a cross-linked polyallylamine or an acid addition salt thereof which is obtained by copolymerization of allylammonium dihydrogen phosphate with an acid addition salt of *N,N'*-diallyl-1,3-diaminopropane in an amount of 5 to 25 mol% with respect to the amount of the allylammonium dihydrogen phosphate, the cross-linked polyallylamine or an acid addition salt thereof having:

a phosphate adsorption capacity of 2.7 to 5.0 mmol/g; and
a degree of swelling of 2.0 to 5.0.

The phosphate adsorption capacity of the above-described cross-linked polyallylamine or an acid addition salt thereof is preferably 2.7 to 4.8 mmol/g, and more preferably 2.7 to 4.5 mmol/g.

The above-described cross-linked polyallylamine or an acid addition salt thereof is preferably given surface cross-linking treatment by reacting a compound having 2 or more amino group-reactive functional groups with an amino group.

The above-described compound having 2 or more amino group-reactive functional groups is preferably an acrylic acid ester, methacrylic acid ester, epihalohydrin, dihalogenated hydrocarbon, diepoxide or dibasic acid chloride, more preferably an acrylic acid ester.

The acid addition salt of the above-described *N,N'*-diallyl-1,3-diaminopropane is preferably *N,N'*-diallyl-1,3-diaminopropane bis(dihydrogen phosphate).

The present invention also provides a pharmaceutical composition comprising as an effective ingredient the above-described cross-linked polyallylamine or an acid addition salt thereof, and further provides a therapeutic or prophylactic agent for hyperphosphatemia, comprising as an effective ingredient the above-described cross-linked polyallylamine or an acid addition salt thereof. The present invention further provides a therapeutic or prophylactic method for hyperphosphatemia, comprising administration of an effective amount of the above-described cross-linked polyallylamine or an acid addition salt thereof to a patient for whom therapy or prophylaxis of hyperphosphatemia is desired. The present invention still further provides use of the above-described cross-linked polyallylamine or an acid addition salt thereof, for the production of a therapeutic or prophylactic agent for hyperphosphatemia. The present invention still further provides a compound for therapy or prophylaxis of hyperphosphatemia, which is the above-described cross-linked polyallylamine or an acid addition salt thereof.

## EFFECT OF THE INVENTION

According to the present invention, a cross-linked polyallylamine or an acid addition salt thereof, which has both a high phosphate adsorption capability and a low degree of swelling can be provided. Further, the cross-linked polyallylamine or the acid addition salt thereof of the present invention has a high phosphate selectivity and a phosphate adsorption capability not less than that of sevelamer hydrochloride, and its degree of swelling is remarkably lower than that of sevelamer hydrochloride, so that, in cases where it is used as a pharmaceutical agent, especially as a therapeutic or prophylactic agent for hyperphosphatemia, administration thereof at a high dose which is required for sufficient inhibition of phosphate absorption is possible and a therapeutic and prophylactic effect can be observed without using a calcium formulation or the like in combination, while reducing side effects such as constipation, abdominal pain and abdominal distension.

## BRIEF DESCRIPTION OF THE DRAWINGS

Fig. 1 shows results of the urinary phosphate excretion test in Example 5.
Fig. 2 shows results of the urinary phosphate excretion test in Example 6.
Fig. 3 shows results of the urinary phosphate excretion test in Example 22.
Fig. 4 shows results of the urinary phosphate excretion test in Example 23.
Fig. 5 shows results of the urinary phosphate excretion test in Example 24.
Fig. 6 shows results of the test for adhesion to the intestinal tract in Example 25.
Fig. 7 shows results of the test for adhesion to the intestinal tract in Example 26.

## BEST MODE FOR CARRYING OUT THE INVENTION

The cross-linked polyallylamine of the present invention or an acid addition salt thereof is obtained by copolymerization of allylammonium dihydrogen phosphate with an acid addition salt of *N,N'*-diallyl-1,3-diaminopropane in an amount of 5 to 25 mol% with respect to the amount of the allylammonium dihydrogen phosphate, the cross-linked polyallylamine or an acid addition salt thereof having:

a phosphate adsorption capacity of 2.7 to 5.0 mmol/g; and
a degree of swelling of 2.0 to 5.0.

To obtain the above-described cross-linked polyallylamine or an acid addition salt thereof, allyl ammonium dihydrogen phosphate is used as a monomer. To maintain a high phosphate adsorption capacity while suppressing the swelling, it is important to use as the monomer allylammonium dihydrogen phosphate which is the phosphate of allylamine. This is because, for example, in cases where allylammonium chloride is used as the monomer, the phosphate adsorption capacity of the obtained cross-linked polyallylamine or an acid addition salt thereof is very low. Allylammonium dihydrogen

phosphate may be prepared in advance from allylamine and phosphoric acid, or allylamine and phosphoric acid may be mixed in a reaction vessel as they are to carry out polymerization reaction.

The above-described cross-linking agent means a polyfunctional allylamine derivative having 2 or more functional groups capable of copolymerizing with allylammonium dihydrogen phosphate.

To obtain the above-described cross-linked polyallylamine or an acid addition salt thereof, an acid addition salt of an N,N-diallyl-substituted allcylenediamine is used as the cross-linking agent. To obtain a cross-linked polyallylamine or an acid addition salt thereof having a low degree of swelling and a high phosphate adsorption capacity, it is important to use as the cross-linking agent an acid addition salt of *N,N'*-diallyl-1,3-diaminopropane. For example, in cases where *N,N'*-diallyl-1,2-diaminoethane, *N,N'*-diallyl-1,4-diaminobutane or *N,N'*-diallyl-1,5-diaminopentane is used, the phosphate adsorption capacity of the obtained polymer is low or the degree of swelling thereof is high.

Examples of the acid added to the above-described *N,N'*-diallyl-1,3-diaminopropane include inorganic acids such as hydrochloric acid, phosphoric acid and sulfuric acid; and organic acids such as formic acid and acetic acid. Hydrochloric acid, phosphoric acid and sulfuric acid are preferred, and phosphoric acid is most preferred. The acid addition salt of *N,N'*-diallyl-1,3-diaminopropane may be prepared in advance from *N,N'*-diallyl-1,3-diaminopropane and an acid, or *N,N'*-diallyl-1,3-diaminopropane and the acid may be mixed in a reaction vessel as they are to carry out polymerization reaction.

The amount of the above-described acid addition salt of *N,N'*-diallyl-1,3-diaminopropane to be added is 5 to 25 mol%, more preferably 10 to 25 mol% and most preferably 15 to 25 mol% with respect to the amount of the monomer, allylammonium dihydrogen phosphate. With the amount of less than 5 mol%, the degree of swelling of the obtained polymer is high, so that the obtained polymer is not preferably used as an oral phosphate adsorption agent. In cases where the amount of the cross-linking agent to be added is 5 to 25 mol%, a polymer having a degree of swelling of not more than 5.0 is obtained, which degree of swelling is much lower than 6.2, the degree of swelling of sevelamer hydrochloride.

The phosphate adsorption capacity of the above-described cross-linked polyallylamine or an acid addition salt thereof is not less than 2.7 mmol/g, preferably not less than 2.8 mmol/g, more preferably not less than 3.0 mmol/g and still more preferably not less than 4.0 mmol/g. The upper limit of the phosphate adsorption capacity is 5.0 mmol/g, preferably 4.8 mmol/g and more preferably 4.5 mmol/g. As indicated in Examples, since the maximum phosphate adsorption capacity of sevelamer hydrochloride is 2.7 mmol/g, the above-described cross-linked polyallylamine or an acid addition salt thereof has a phosphate adsorption capacity not less than that of sevelamer hydrochloride. The phosphate adsorption capacity means the amount of phosphate ions removed by adsorption to a sample to be measured in a test solution containing phosphate and glycocholate in a molar ratio of 1:1. More particularly, the measurement is carried out as follows. That is, a sample to be measured is stirred in 50 mM hydrochloric acid at 37°C for 1 hour, and each of disodium hydrogen phosphate dodecahydrate and an aqueous sodium glycocholate solution is added to a final concentration of 10 mM, such that the final concentration of the sample to be measured becomes 1 mg/mL in the solution. After 1 hour of stirring at 37°C, centrifugation (refrigerated centrifuge 5417R manufactured by Eppendorf, angle rotor FA-45-24-11) is carried out at conditions of 15,000 rpm, 25°C and 15 minutes to remove the sample to be measured, and the amount of phosphate which was not adsorbed to the sample to be measured is measured (n=3) using an inorganic phosphate measurement reagent (manufactured by Wako Pure Chemical Industries; Phosphor C (registered trademark)), to determine the amount of the phosphate ions adsorbed to the sample to be measured, that is, the phosphate adsorption capacity (a calibration curve was prepared within the range of the inorganic phosphate concentration of 0 to 386 mg/mL; the spectrophotometer is Spectra Max Plus manufactured by Molecular Devices). In the assay method described in the above-described Patent Literature 1 (JP 3113283 A), the phosphate adsorption capacity was measured in the presence of phosphate alone without adding a bile acid such as glycocholate, but, since bile acids represented by glycocholate are present in large amounts in the intestine of the body and compete with phosphate in terms of adsorption, the assay in the present invention is made based on the phosphate adsorption capacity under the bile acid-competing condition as described above.

The cross-linked polyallylamine or the acid addition salt thereof of the present invention has a degree of swelling of 2.0 to 5.0. In cases where the degree of swelling is higher than 5.0, there is a possibility that the risk of side effects due to swelling is high. The range of the degree of swelling is preferably 2.0 to 4.5, more preferably 2.0 to 4.0, still more preferably 2.0 to 3.5. Since the degree of swelling of sevelamer hydrochloride is 6.2 as indicated in the Examples, less side effects due to swelling are expected in the cross-linked polyallylamine or the acid addition salt thereof of the present invention than sevelamer hydrochloride. The degree of swelling means a value obtained by soaking 200 mg of a sample to be measured, which was dried under reduced pressure at 40°C for not less than 16 hours, in 50 mL of distilled water for not less than 24 hours and filtrating the resultant using a membrane filter (Omnipore, manufactured by Millipore) having a diameter of 47 mm and a pore size of 0.45 $\mu$m under reduced pressure to separate the solid component, whose weight is then divided by the dry weight (200 mg).

To further decrease the degree of swelling of the cross-linked polyallylamine or the acid addition salt thereof of the present invention, there is a method to increase the crosslink density in the vicinity of the surfaces of particles of

the cross-linked polyallylamine or an acid addition salt thereof by a surface cross-linking treatment wherein a compound having 2 or more amino group-reactive functional groups (hereinafter referred to as "surface cross-linking agent") is allowed to react with amino groups of the cross-linked polyallylamine or an acid addition salt thereof.

Examples of the surface cross-linking agent used in the present invention include acrylic acid esters, methacrylic acid esters, epihalohydrins, dihalogenated hydrocarbons, diepoxides and dibasic acid chlorides. Acrylic acid esters, epihalohydrins and dihalogenated hydrocarbons are preferred; acrylic acid esters and epihalohydrins are more preferred; and acrylic acid esters are most preferred.

Examples of the acrylic acid esters include methyl acrylate, ethyl acrylate, propyl acrylate, isopropyl acrylate, butyl acrylate, isobutyl acrylate, *sec*-butyl acrylate, 2-hydroxyethyl acrylate, 2-hydroxypropyl acrylate and glycidyl acrylate. Methyl acrylate, ethyl acrylate and 2-hydroxyethyl acrylate are preferred; methyl acrylate and 2-hydroxyethyl acrylate are more preferred; and 2-hydroxyethyl acrylate is most preferred.

Examples of the methacrylic acid esters include methyl methacrylate, ethyl methacrylate, propyl methacrylate, isopropyl methacrylate, butyl methacrylate, isobutyl methacrylate, *sec*-butyl methacrylate, 2-hydroxyethyl methacrylate, 2-hydroxypropyl methacrylate and glycidyl methacrylate.

Examples of the epihalohydrins include epichlorohydrin and epibromohydrin; and epichlorohydrin is preferred.

Examples of the dihalogenated hydrocarbons include 1,2-dichloroethane, 1,2-dibromoethane, 1,3-dichloropropane, 1,3-dibromopropane, 1,4-dichlorobutane, 1,4-dibromobutane, 1,4-dichloro-2-butene and 3,4-dichloro-1-butene.

Examples of the diepoxides include 1,2,3,4-diepoxybutane, 1,2-ethanediol diglycidyl ether and 1,4-butanediol diglycidyl ether.

Examples of the dibasic acid chlorides include oxalyl chloride, malonyl chloride, succinyl chloride, glutaryl chloride and adipoyl chloride.

The cross-linked polyallylamine or an acid addition salt thereof obtained in the present invention by surface cross-linking has a phosphate adsorption capacity of not less than 2.7 mmol/g, preferably not less than 2.8 mmol/g, more preferably not less than 3.0 mmol/g, still more preferably not less than 4.0 mmol/g. The upper limit of the phosphate adsorption capacity is 5.0 mmol/g, preferably 4.8 mmol/g, more preferably 4.5 mmol/g. As indicated in the Examples, since the phosphate adsorption capacity of sevelamer hydrochloride is 2.7 mmol/g, the cross-linked polyallylamine or an acid addition salt thereof obtained in the present invention by surface cross-linking has a phosphate adsorption capacity not less than that of sevelamer hydrochloride.

The cross-linked polyallylamine or an acid addition salt thereof obtained in the present invention by surface cross-linking has a degree of swelling of 2.0 to 5.0. In cases where the degree of swelling is higher than 5.0, there is a possibility that the risk of side effects due to swelling is high. The range of the degree of swelling is preferably 2.0 to 4.5, more preferably 2.0 to 4.0, still more preferably 2.0 to 3.5. Since the degree of swelling decreases by surface cross-linking, the side effects are considered to be reduced.

A higher selectivity to phosphate than to bile acids is one of the characteristics of the cross-linked polyallylamine or the acid addition salt thereof of the present invention. The phosphate selectivity is preferably not less than 1.5, more preferably not less than 2.0, still more preferably not less than 2.5. The upper limit is not limited, and a larger value is preferred, but it is not more than 10 in the present invention. The phosphate selectivity in the present invention means a value obtained by dividing the phosphate adsorption capacity by the bile acid adsorption capacity. The bile acid adsorption capacity means the amount of glycocholate ions removed by adsorption to a sample to be measured in a test solution containing phosphate and glycocholate in a molar ratio of 1:1. More particularly, the measurement is carried out as follows. A sample to be measured is stirred in 50 mM hydrochloric acid at 37°C for 1 hour, and each of disodium hydrogen phosphate dodecahydrate and an aqueous sodium glycocholate solution is added to a final concentration of 10 mM, such that the final concentration of the sample to be measured becomes 1 mg/mL in the solution. After 1 hour of stirring at 37°C, centrifugation (refrigerated centrifuge 5417R manufactured by Eppendorf, angle rotor FA-45-24-11) is carried out at conditions of 15,000 rpm, 25°C and 15 minutes to remove the sample to be measured, and the amount of glycocholate which was not adsorbed to the sample to be measured is measured (n=3) using a bile acid measurement reagent (manufactured by Wako Pure Chemical Industries; Total Bile Acids (registered trademark)), to determine from the measured value the amount of the phosphate ions adsorbed to the sample to be measured, that is, the phosphate adsorption capacity (a calibration curve was prepared within the range of the inorganic phosphorus concentration of 0 to 386 mg/mL; the spectrophotometer is Spectra Max Plus manufactured by Molecular Devices).

Thus, the cross-linked polyallylamine or the acid addition salt thereof of the present invention has excellent characteristics as an oral phosphate adsorption agent, which cannot be obtained by the method described in the above-described Patent Literature 2 (JP 10-330427 A). Further, since it has a higher phosphate adsorption capacity and a lower degree of swelling than sevelamer hydrochloride, side effects caused by excessive swelling which are problematic in a medical use of sevelamer hydrochloride can be reduced. The side effect-reducing effect can be confirmed by observing in an animal experiment the moving speed of the content of the intestinal tract, excretion speed thereof, and the amount thereof adhered to the intestinal tract.

Further, the cross-linked polyallylamine or the acid addition salt thereof of the present invention causes less

adherence to the intestinal tract than sevelamer hydrochloride. Since adherence of a polymer or the like to the intestinal tract may be a factor inhibiting the movement of the intestine, it is thought that side effects of the gastrointestinal tract such as constipation are smaller in the cross-linked polyallylamine or the acid addition salt thereof of the present invention than in sevelamer hydrochloride.

Examples of a method which can be used for polymerization for production of the cross-linked polyallylamine or the acid addition salt thereof of the present invention include known methods such as solution polymerization, reversed phase suspension polymerization, emulsion polymerization and precipitation polymerization. Examples of the solvent in which monomers are allowed to dissolve upon the polymerization include water; aqueous solutions of inorganic acids such as hydrochloric acid, phosphoric acid and sulfuric acid, and organic acids such as formic acid and acetic acid; polar solvents such as methanol, ethanol, 1-propanol, 2-propanol, *N,N*-dimethylformamide and dimethylsulfoxide; and mixed solvents wherein 2 or more of these solvents are arbitrarily mixed, and, among these, water and mixed solvents of water and a polar solvent are preferred. The amount of the solvent is preferably 0.2 mL to 3.0 mL, more preferably 0.3 mL to 2.0 mL, most preferably 0.3 mL to 1.5 mL with respect to 1 g of the total weight of monomer allylammonium dihydrogen phosphate and the cross-linking agent.

In the polymerization condition of a two-phase system such as reversed phase suspension polymerization or emulsion polymerization, known organic solvents can be used as a dispersion medium which disperses the monomer solution, and examples thereof include hexane, cyclohexane, heptane, octane, decane, petroleum ether, liquid paraffin, ethyl acetate, propyl acetate and isopropyl acetate; and mixed solvents wherein 2 or more of these solvents are arbitrarily mixed. Cyclohexane, heptane and octane are preferred; heptane and octane are more preferred; and heptane is most preferred.

In the polymerization condition of a two-phase system such as reversed phase suspension polymerization and emulsion polymerization, a surfactant is added as required. Examples of the surfactant which may be used include sorbitan monolaurate, sorbitan monooleate, sorbitan monostearate, sorbitan monopalmitate, ethylene glycol monostearate, glyceryl monostearate, polyethylene glycol monostearate, polyethylene glycol hydrogenated castor oil, polyoxyethylene sorbitan monolaurate, polyoxyethylene sorbitan monooleate, polyethylene glycol and diisooctyl sulfosuccinate. Sorbitan monolaurate, sorbitan monooleate, sorbitan monostearate and sorbitan monopalmitate are more preferred; and sorbitan monolaurate is most preferred.

To form macropores in the obtained polymer, a diluent such as methanol, ethanol, 1,1-dimethylethanol, octanol, 2-propanol and hexane may be added, but addition of the above-described diluent is not preferred.

As the polymerization initiator, an azo radical initiator is used. Examples of the azo radical initiator which may be used include known azo radical initiators such as 2-cyano-2-propylazoformamide, 1,1'-azobis(cyclohexane-1-carbonitrile), 2,2'-azobis(2-amidinopropane) dihydrochloride, 2,2'-azobis(2-methylbutyronitrile), 2,2'-azobisisobutyronitrile, 2,2'-azobis(2,4-dimethylvaleronitrile), 2,2'-azobis(4-methoxy-2,4-dimethylvaleronitrile), 4,4'-azobis(4-cyanovaleric acid), dimethyl 2,2'-azobisisobutyrate, 2,2'-azobis[2-(2-imidazolin-2-yl)propane] dihydrochloride, 2,2'-azobis[2-(2-imidazolin-2-yl)propane] disulfate dihydrate, 2,2'-azobis[N-(2-carboxyethyl)-2-methylpropionamide], 2,2'-azobis{2-[1-(2-hydroxyethyl)-2-imidazolin-2-yl]propane} dihydrochloride, 2,2'-azobis[2-(2-imidazolin-2-yl)propane], 2,2'-azobis(1-imino-1-pyrrolidino-2-methylpropane) dihydrochloride, 2,2'-azobis{2-methyl-N-[1,1-bis(hydroxymethyl)-2-hydroxyethyl]propionamide), 2,2'-azobis[2-methyl-N-(2-hydroxyethyl)propionamide], 2,2'-azobis(N-butyl-2-methylpropionamide), 2,2'-azobis (N-cyclohexyl-2-methylpropionamide), dimethyl 1,1'-azobis(1-cyclohexanecarboxylate), 2,2'-azobis[N-(2-propenyl)-2-methylpropionamide] and 2,2'-azobis(2,4,4-trimethylpentane). 2,2'-azobis(2-amidinopropane)dihydrochloride, 4,4'-azobis(4-cyanovaleric acid), 2,2'-azobis[2-(2-imidazolin-2-yl)propane] dihydrochloride, 2,2'-azobis[2-(2-imidazolin-2-yl)propane] disulfate dihydrate, 2,2'-azobis{2-[1-(2-hydroxyethyl)-2-imidazolin-2-yl]propane} dihydrochloride and 2,2'-azobis (1-imino-1-pyrrolidino-2-methylpropane) dihydrochloride are preferred; 2,2'-azobis(2-amidinopropane) dihydrochloride, 2,2'-azobis[2-(2-imidazolin-2-yl)propane] dihydrochloride and 2,2'-azobis[2-(2-imidazolin-2-yl)propane] disulfate dihydrate are more preferred; and 2,2'-azobis(2-amidinopropane) dihydrochloride and 2,2'-azobis[2-(2-imidazolin-2-yl)propane] dihydrochloride are most preferred.

The amount of the initiator to be added is preferably 0.1 mol% to 10 mol%, more preferably 0.5 mol% to 8 mol%, most preferably 0.7 mol% to 5 mol% with respect to the total number of moles of the monomers and the cross-linking agent.

The polymerization temperature is preferably 30°C to 100°C, more preferably 40°C to 80°C, most preferably 40°C to 70°C. The polymerization time varies depending on the polymerization temperature and the like, and not more than 100 hours is usually sufficient.

By allowing an acid addition salt of *N,N'*-diallyl-1,3-diaminopropane having two terminal double bonds to coexist when the radical polymerization of allylammonium dihydrogen phosphate is carried out, the polymer strand is elongated at these both termini to form a cross-linking structure. During this, in a part of the cross-linking agent, one of the double bonds may not react and remain as the double bond without being involved in cross-linking.

When the production is carried out as described above, the cross-linked polyallylamine or the acid addition salt thereof of the present invention having a phosphate adsorption capacity and a degree of swelling within the above-described ranges can be obtained. However, depending on reaction conditions (concentration of the reactant, reaction temperature, reaction time and the like) used, properties (phosphate adsorption capacity, degree of swelling and phosphate selectivity) of the obtained cross-linked polyallylamine polymer may not fall within the above-described ranges. In this case, the properties can be adjusted to fall within the above-described ranges by changing reaction conditions as appropriate. Further, even in cases where the properties of the obtained cross-linked polyallylamine polymer are within the above-described ranges, reaction conditions may be changed in order to obtain a cross-linked polyallylamine polymer having better properties. The relationship between reaction conditions and properties of the obtained cross-linked polymer is shown in Example 1 below. Concrete examples of the method for changing reaction conditions for adjustment of the properties include, but are not limited to: (1) increase of the concentration of the reactant during polymerization reaction (decrease of the solvent volume); (2) lowering of the polymerization temperature and extension of the reaction time; (3) use of a larger amount of the radical initiator. In cases where (1) is carried out, the phosphate adsorption capacity of the obtained cross-linked polyallylamine polymer slightly decreases, but the degree of swelling decreases, which is preferred. In cases where (2) is carried out, the phosphate adsorption capacity slightly decreases, but the phosphate selectivity is improved and the degree of swelling decreases, which are preferred. In cases where (3) is carried out, the phosphate adsorption capacity slightly decreases, but the degree of swelling decreases, which is preferred. Further, in cases where the purities of the monomers and the initiator are increased, the radical termination reaction is suppressed, so that the degree of swelling decreases. Further, in cases where the diameter of the obtained cross-linked polyallylamine or an acid addition salt thereof is decreased, the phosphate adsorption capacity is improved.

Since the cross-linked polyallylamine obtained by the polymerization reaction is a phosphate, it needs to be converted to the free form or another acid addition salt in order to use it as a phosphate adsorption agent. The conversion to the free form is carried out by washing with water or neutralization. Examples of the alkali used for the neutralization include inorganic bases such as sodium hydroxide, potassium hydroxide, lithium hydroxide, barium hydroxide, sodium hydrogen carbonate, potassium carbonate and ammonia; and metal alkoxides such as sodium methoxide, sodium ethoxide and potassium tert-butoxide.

In cases where the free-form cross-linked polyallylamine is converted to an acid addition salt, a pharmaceutically acceptable acid other than phosphoric acid can be used, and examples thereof include hydrochloric acid, hydrobromic acid, hydroiodic acid, sulfuric acid, nitric acid, carbonic acid, acetic acid, benzoic acid, succinic acid, tartaric acid and citric acid. Hydrochloric acid, carbonic acid and acetic acid are more preferred; and hydrochloric acid and acetic acid are most preferred. The amount of the acid may be an arbitrary amount not more than 1 equivalent with respect to the amino group of the cross-linked polyallylamine, and 0.1 equivalent to 0.8 equivalent is more preferred, and 0.1 equivalent to 0.5 equivalent is most preferred. Further, by allowing an excess amount of the acid to act on the phosphate of the cross-linked polyallylamine obtained by the polymerization, it can be converted to the acid addition salt of interest without conversion to the free form. Conversion to the free form is preferably carried out by a method wherein the phosphate of the polymer is allowed to react with an aqueous sodium hydroxide solution and the phosphate is removed by filtration, followed by stirring in water, washing and filtration. The cross-linked polyallylamine may also be used as the free form without conversion to an acid addition salt.

The reaction of the surface cross-linking in the present invention is carried out with a cross-linked polyallylamine or an acid addition salt thereof dispersed in the solvent, and the reaction is preferably carried out with a cross-linked polyallylamine of the free form.

The amount of the surface cross-linking agent to be added is selected as appropriate within the range of 1.0% by weight to 50% by weight with respect to the weight of the cross-linked polyallylamine or an acid addition salt thereof to be subjected to surface cross-linking. The amount of 1.5% by weight to 40% by weight is preferred, 2.5% by weight to 20% by weight is more preferred, and 2.5% by weight to 10% by weight is most preferred. The larger the amount of the surface cross-linking agent to be added, the lower the degree of swelling of the obtained polymer, but addition of an excess amount thereof is not preferred since the phosphate adsorption capacity decreases thereby.

The solvent used for the surface cross-linking is selected as appropriate depending on the surface cross-linking agent. Examples of the solvent which may be used include alcoholic solvents represented by methanol, ethanol, 1-propanol and 2-propanol; hydrocarbon solvents represented by hexane, heptane, cyclohexane and toluene; known solvents such as ethyl acetate, tetrahydrofuran, acetone, acetonitrile, *N,N*-dimethylformamide, dimethylsulfoxide and water; and mixed solvents wherein 2 or more of these solvents are arbitrarily mixed.

The temperature for the surface cross-linking is selected as appropriate depending on the surface cross-linking agent, and preferably 0°C to 80°C, more preferably 20°C to 60°C, most preferably 20°C to 50°C.

The time for the surface cross-linking is selected as appropriate depending on the surface cross-linking agent and the temperature, and usually preferably 5 minutes to 10 hours.

During the surface cross-linking, the cross-linking reaction occurs by reaction of the surface cross-linking agent with two amino groups existing in the vicinity of the surface of particles of the cross-linked polyallylamine or an acid addition salt thereof. Scheme 1 shows explanation of an example of a reaction wherein acrylic acid ester (2-hydroxyethyl acrylate) is used as the surface cross-linking agent. First, Michael addition of an amino group on the surface of a polymer

particle to the acrylic acid ester is allowed to occur (Step 1). Subsequently, an amino group in the vicinity on the surface of the particle attacks the carbonyl group of the ester to allow amidation to proceed (Step 2). By this, cross-linking in the vicinity of the surface of the polymer particle is strengthened. During this, depending on reaction conditions, only Step 1 proceeds and cross-linking may not occur in a part of the surface cross-linking agent.

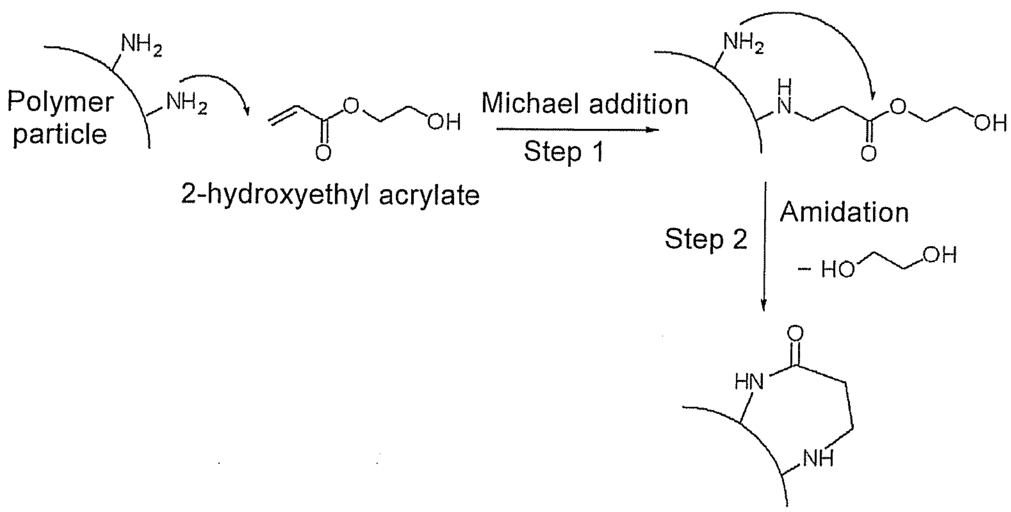


Scheme 1

After the surface cross-linking, the cross-linked polyallylamine may be salified to an acid addition salt as required. The conversion to an acid addition salt is carried out in the same manner as described above. Examples of the acid used for the salt formation include pharmaceutically acceptable acids such as hydrochloric acid, hydrobromic acid, hydroiodic acid, sulfuric acid, nitric acid, carbonic acid, acetic acid, benzoic acid, succinic acid, tartaric acid and citric acid; and hydrochloric acid, carbonic acid and acetic acid are more preferred; and hydrochloric acid and acetic acid are most preferred. The amount of the acid may be an arbitrary amount not more than 1 equivalent with respect to the amino group of the polymer, and 0.1 equivalent to 0.8 equivalent is preferred, and 0.1 equivalent to 0.5 equivalent is more preferred. The polymer obtained in the present invention is subjected to pulverization as required. The method of pulverization is not limited, and either dry or wet pulverization may be carried out.

The cross-linked polyallylamine or the acid addition salt thereof of the present invention can be used as a pharmaceutical composition because of its high phosphate adsorption capacity and low degree of swelling, and can be preferably used as especially a therapeutic or prophylactic agent for hyperphosphatemia. In this case, by orally administering the cross-linked polyallylamine or the acid addition salt thereof of the present invention as it is in the form of powder or as a medical composition in an appropriate formulation to a mammal, phosphates in the intestine is adsorbed to the cross-linked polyallylamine or an acid addition salt thereof, whereby contributing to therapy or prophylaxis of hyperphosphatemia.

Examples of the formulation of the pharmaceutical composition containing as an effective component the cross-linked polyallylamine or the acid addition salt thereof of the present invention include tablets, powders, pills, capsules and granules. The above-described formulations are produced by known methods and may contain various carriers normally used in the field of drug formulation. Examples of the various carriers include fillers, lubricants, binders and disintegrators. In addition, additives such as antiseptics, antioxidants, coloring agents, sweeteners, adsorbents and wetting agents may be used as required.

Examples of the fillers include lactose, D-mannitol, potato starch, sucrose, corn starch, crystalline cellulose and light anhydrous silicic acid.

Examples of the lubricants include magnesium stearate, calcium stearate, talc and colloidal silica.

Examples of the binders include crystalline cellulose, D-mannitol, dextrin, hydroxypropyl cellulose, hydroxypropylmethyl cellulose, polyvinylpyrrolidone, starch, sucrose, methyl cellulose and sodium carboxymethyl cellulose.

Examples of the disintegrators include starch, carboxymethyl cellulose, calcium carboxymethyl cellulose, croscarmellose sodium, sodium carboxymethyl starch and low substituted hydroxypropylcellulose.

Examples of the antiseptics include p-oxybenzoic acid esters, chlorobutanol, benzylalcohol, phenetyl alcohol, dehydroacetic acid and sorbic acid.

Examples of the antioxidants include sulfurous acid salts and ascorbic acid.

The effective dose and the number of doses of the pharmaceutical composition containing as an effective component the cross-linked polyallylamine or the acid addition salt thereof of the present invention vary depending on the dosage form, and age, body weight and severity of the symptom of the patient, and usually 0.1 to 15 g, preferably 0.5 to 9 g of the pharmaceutical composition may be administered to an adult per day before, during or after eating.

The pharmaceutical composition containing as an effective component the cross-linked polyallylamine or the acid addition salt thereof of the present invention may be administered solely or in combination with oral phosphate adsorption agents such as calcium carbonate, calcium lactate, calcium acetate, magnesium oxide and lanthanum carbonate.

EXAMPLES

The present invention will now be described in more detail by way of Examples below, but the present invention is not limited to these Examples.

(Reference Example 1)

Synthesis of Allylammonium Dihydrogen Phosphate

To a three-necked round bottom flask equipped with a mechanical stirrer and a thermometer, 46.1 g (0.40 mol) of phosphoric acid (85%) and 500 mL of ethanol were placed, and 30.0 mL (0.40 mol) of allylamine was added thereto with ice cooling. After 30 minutes of stirring at room temperature, precipitated white crystals were recovered by filtration and washed with ethanol sufficiently. After drying the recovered crystals under reduced pressure at 60°C, 59.6 g of allylammonium dihydrogen phosphate was obtained.

(Reference Example 2)

Synthesis of *N,N'*-diallyl-1,3-diaminopropane and Its Bis(dihydrogen phosphate)

To a flask, 38.0 mL (0.40 mol) of 1,3-dichloropropane and 300 mL (4.00 mol) of allylamine were placed, and the resulting mixture was heated with stirring under argon atmosphere at 50 to 52°C for 16 hours. About a half amount of the excessive allylamine was evaporated under reduced pressure, and an aqueous potassium hydroxide solution (prepared by dissolving 48 g of potassium hydroxide into 144 g of water) was added thereto. Precipitated salt was removed by filtration, and the filtrate was concentrated under reduced pressure to about a half volume. After extraction with diethylether, the organic layer was dried over anhydrous sodium sulfate and concentrated under reduced pressure, to obtain an oily crude product. This crude product was distilled under reduced pressure (42 to 44°C/0.3 kPa) to obtain 38.4 g of *N,N'*-diallyl-1,3-diaminopropane. To a flask, 15.0 g (130 mmol) of phosphoric acid (85%) and 300 mL of ethanol were placed, and 10.0 g (65.0 mmol) of *N,N'*-diallyl-1,3-diaminopropane dissolved in 20 mL of ethanol with ice cooling was added thereto. After 30 minutes of stirring at room temperature, precipitated white crystals were recovered by filtration and washed with ethanol. After drying the recovered crystals under reduced pressure at 60°C, 22.6 g of *N,N'*-diallyl-1,3-diaminopropane bis(dihydrogen phosphate) was obtained. The total amount thereof was suspended in 175 mL of methanol and dissolved by adding 14 mL of water while heating the mixture to reflux. The resulting solution was stirred at room temperature, thereby precipitating crystals. The resultant was left to stand in a freezer at -10°C for 1 hour and filtered, followed by washing the precipitate with ice-cooled methanol/water (100/2 v/v). Subsequently, the precipitate was washed with ethanol and dried under reduced pressure at 60°C to obtain 22.0 g of *N,N'*-diallyl-1,3-diaminopropane bis(dihydrogen phosphate).

(Reference Example 3)

Synthesis of *N,N'*-diallyl-1,2-diaminoethane and Its Bis(dihydrogen phosphate)

To a flask, 7.9 mL (0.10 mol) of 1,2-dichloroethane and 75 mL (1.0 mol) of allylamine were placed and the resulting mixture was heated with stirring under argon atmosphere at 50 to 52°C for 20 hours. About a half amount of the excessive allylamine was evaporated under reduced pressure, and an aqueous potassium hydroxide solution (prepared by dissolving 12 g of potassium hydroxide into 36 g of water) was added thereto. Precipitated salt was dissolved by addition of water, and the resulting solution was extracted with diethylether. The organic layer was dried over sodium sulfate and concentrated under reduced pressure to obtain an oily crude product. This crude product was distilled under reduced pressure (85 to 87°C/1.2 kPa) to obtain 8.08 g of *N,N'*-diallyl-1,2-diaminoethane.

To 300 mL of ethanol, 8.41 g (60.0 mmol) of *N,N'*diallyl-1,2-diaminoethane was dissolved. After cooling the

resulting solution to -78°C, 13.8 g (120 mmol) of phosphoric acid (85%) dissolved to 120 mL of ethanol was slowly added dropwise thereto. After 30 minutes of stirring, precipitated white crystals were recovered by filtration and washed with ethanol. After drying the recovered crystals under reduced pressure at room temperature, 19.9 g of *N,N'*diallyl-1,2-diaminoethane bis(dihydrogen phosphate) was obtained. A 15.0 g aliquot of the obtained *N,N'* diallyl-1,2-diaminoethane bis(dihydrogen phosphate) was suspended in 120 mL of methanol and allowed to dissolve by addition of 12 mL of water while heating the mixture to reflux. The resulting solution was stirred at -20°C, thereby precipitating crystals. The resulting precipitate was filtered and washed with ice-cooled methanol/water (100/4 v/v). Subsequently, the precipitate was washed with ethanol and dried under reduced pressure at room temperature to obtain 10.7 g *of N,N*-diallyl-12-diaminoethane bis(dihydrogen phosphate).

(Reference Example 4)

Synthesis of *N,N'*diallyl-1,4-diaminobutane and Its Bis(dihydrogen phosphate)

To 230 mL of acetonitrile, 20.0 g (227 mmol) of 1,4-diaminobutane and 63.3 mL (454 mmol) of triethylamine were dissolved, and 99.0 g (454 mmol) of di-*tert*-butyl dicarbonate was slowly added thereto with ice cooling with stirring. The resulting mixture was stirred at room temperature for 1 hour and cooled to -20°C. The precipitated solids were recovered by filtration and dried under reduced pressure to obtain 47.1 g of a crude product of tert-butyl butane-1,4-diyldicarbamate. This was dissolved to 300 mL of acetonitrile at 60°C and left to stand at room temperature, thereby precipitating needle crystals. After cooling at -20°C, the crystals were recovered by filtration to obtain 45.6 g of di-*tert*-butyl butane-1,4-diyldicarbamate.

In 100 mL of *N,N*-dimethylformamide, 21.0 g (72.9 mmol) of di-*tert*-butyl butane-1,4-diyldicarbamate was suspended. The atmosphere in the flask was replaced with argon, and 18.9 mL (219 mmol) of allyl bromide was added thereto. At an inner temperature of 40°C, 8.74 g (219 mmol) of sodium hydride (60% dispersion in mineral oil) was added slowly thereto. Thereafter, the resulting mixture was stirred while adjusting the inner temperature such that a temperature of 10°C to 20°C is attained in an ice bath. After confirming that there is no foaming and elevation of temperature, 2.91 g (73 mmol) of sodium hydride (60% dispersion in mineral oil) and 6.3 mL (73 mmol) of allyl bromide were added to the mixture, and the resulting mixture was further stirred at room temperature for 2 hours. The mixture was cooled on ice, and water was slowly added thereto, followed by extraction twice with ethyl acetate. After washing the organic layer twice with saturated aqueous ammonium chloride solution, 3 times with water and twice with saturated brine, it was dried over magnesium sulfate. The resultant was concentrated under reduced pressure by a rotary evaporator to obtain a crude product of di-*tent*-butyl butane-1,4-diylbis(allylcarbamate) as an oily product. The total amount of this oily product was dissolved in 220 mL of ethanol, and 50 mL of 6 M hydrochloric acid was added thereto, followed by heating the mixture to reflux for 2.5 hours. After removal of the most part of the oil floating on the liquid, concentration was carried out under reduced pressure. After repeating the operation of adding 100 mL of ethanol to concentrate the solution 3 times, white crystals were precipitated. The resultant was cooled on ice and filtered, followed by washing with ice-cooled ethanol. The resultant was dried under reduced pressure at room temperature to obtain 14.8 g of *N,N'*-diallyl-1,4-diaminobutane dihydrochloride. Its total amount was dissolved in 100 mL of water and 30 mL of 20 w/w% aqueous sodium hydroxide solution was added thereto, followed by 3 times of extraction with diethylether. The organic layer was dried over magnesium sulfate and concentrated. The crude product was distilled under reduced pressure (88°C/0.2 kPa) to obtain 8.65 g of *N,N'*-diallyl-1,4-diaminobutane. To a flask, 8.07 g of phosphoric acid (85%) was weighed, and 150 mL of ethanol and 75 mL of 2-propanol were added thereto, followed by cooling with ice. To this, 5.89 g of *N,N'*-diallyl-1,4-diaminobutane dissolved in 25 mL of ethanol was added, thereby precipitating viscous solids. The resulting solids were left to stand at room temperature for 3 days to become solid masses. These masses were crushed and filtered, followed by washing with ethanol and drying under reduced pressure at room temperature, to obtain 12.8 g of *N,N'*-diallyl-1,4-diaminobutane (dihydrogen phosphate). Its total amount was suspended in 200 mL of methanol, and 25 mL of water was added thereto while heating the mixture to reflux. Since a small portion of the solids remained undissolved, hot filtration was carried out and the filtrate was left to stand at room temperature, thereby precipitating white crystals. The resultant was left to stand in a freezer at - 10°C and filtered, followed by washing the precipitate with methanol/ water (100/4 v/v). Subsequently, the precipitate was washed with ethanol and dried under reduced pressure at 50°C, to obtain 23.1 g of *N,N'*-diallyl-1,4-diaminobutane bis(dihydrogen phosphate).

(Reference Example 5)

Synthesis of *N,N'*-diallyl-1,5-diaminopentane and Its Bis(dihydrogen phosphate)

To 80 mL of acetonitrile, 7.85 g (76.8 mmol) of 1,5-diaminopentane and 21.4 mL (154 mmol) of triethylamine were dissolved, and 33.6 g (154 mmol) of di-*tert*- butyl dicarbonate was slowly added thereto with ice cooling with stirring.

The resulting mixture was stirred at room temperature for 1 hour, and concentrated to dryness by a rotary evaporator. To the resultant, 20 mL of ethyl acetate was added, and it was dissolved therein under heat at 60°C, followed by addition of 100 mL of hexane and stirring at room temperature, thereby precipitating crystals. The crystals were recovered by filtration to obtain 16.1 g of di-*tert*-butyl pentane-1,5-diyldicarbamate.

In 200 mL of *N,N*-dimethylformamide, 45.4 g (150 mmol) of di-*tert*-butyl pentane-1,5-diyldicarbamate was suspended. The atmosphere in the flask was replaced with argon, and 38.9 mL (450 mmol) of allyl bromide was added thereto. At an inner temperature of 40°C, 18.0 g (450 mmol) of sodium hydride (60% dispersion in mineral oil) was added slowly thereto. The resulting mixture was cooled on ice, and water was slowly added thereto, followed by 3 times of extraction with ethyl acetate. After washing the organic layer 3 times with water and twice with saturated brine, it was dried over magnesium sulfate. The resultant was concentrated under reduced pressure by a rotary evaporator, to obtain a crude product of di-*tert*-butyl pentane-1,5-diylbis(allylcarbamate) as an oily product. The total amount of this oily product was dissolved in 440 mL of ethanol, and 100 mL of 6M hydrochloric acid was added thereto, followed by heating the mixture to reflux for 1 hour. After repeating the operation of adding 200 mL of ethanol to concentrate the solution twice, white crystals were precipitated. The resulting precipitate was filtered and washed with ethanol. This was dried under reduced pressure at room temperature to obtain 24.5 g of *N,N'*-diallyl-1,5-diaminopentane dihydrochloride.
Its total amount was dissolved in 100 mL of water and 50 g of 20 w/w% aqueous sodium hydroxide solution was added thereto, followed by extraction with diethylether twice. The organic layer was dried over magnesium sulfate and concentrated. The crude product was distilled under reduced pressure (64°C/0.1 kPa) to obtain 7.73 g of *N,N'*-diallyl-1,5-diaminopentane. Into a flask, 9.78 g of phosphoric acid (85%) was weighed, and 160 mL of ethanol was added thereto, followed by cooling with ice. To this, 7.73 g of *N,N'*-diallyl-1,5-diaminopentane dissolved in 40 mL of ethanol was added, thereby precipitating white solids. The resulting solids were left to stand at room temperature for 3 days to become solid masses. These masses were crushed and filtered, followed by washing with ethanol, and drying under reduced pressure at 50°C, to obtain 16.0 g of *N,N'*-diallyl-1,5-diaminopentane dihydrogen phosphate. Its total amount was suspended in 140 mL of methanol, and 2.8 mL of water was added thereto while heating the mixture to reflux. Since a small portion of the solids remained undissolved, hot filtration was carried out and the filtrate was left to stand at room temperature, thereby precipitating white crystals. The resultant was filtered and washed with ice-cooled methanol/water (100/4 v/v). Subsequently, the precipitate was washed with ethanol and dried under reduced pressure at 50°C to obtain 13.1 g of *N,N'*-diallyl-1,5-diaminopentane bis(dihydrogen phosphate).

(Reference Example 6)

Synthesis of Sevelamer Hydrochloride

To a flask, 173 mL of concentrated hydrochloric acid was added, and 120 g (2.10 mol) of allylamine was added dropwise thereto at an inner temperature of 5 to 10°C. After completion of the dropping, 90 mL of the liquid was evaporated under reduced pressure while being heated in an oil bath at 70°C. After replacing the atmosphere in the system with argon 3 times, the mixture was bubbled with argon for 30 minutes. In 5.4 mL of water, 2.40 g (8.85 mmol) of 2,2'-azobis (2-amidinopropane) dihydrochloride was suspended, and the resulting suspension was added to the above mixture, followed by stirring of the resulting mixture at an inner temperature of 50°C for 24 hours. In 5.4 mL of water, 2.40 g (8.85 mmol) of 2,2'-azobis(2-amidinopropane) dihydrochloride was suspended, and the resulting suspension was added to the above mixture, followed by stirring of the resulting mixture at an inner temperature of 50°C for additional 44 hours. To this mixture, 48 mL of water was added , and the resulting mixture was cooled to room temperature and poured slowly to 2L of methanol with stirring. The obtained white solids were filtered. The operation wherein the solids were added to 2L of methanol and the resulting mixture was stirred for 1 hour, followed by filtration thereof was repeated twice. The obtained solids were dried in a vacuum oven at 50°C for 24 hours to obtain 111 g of polyallylamine hydrochloride.

To a 200 mL beaker, 25.0 g of the obtained polyallylamine hydrochloride was weighed, and dissolved in 100 mL of water. To the resulting solution, 7.12 g of sodium hydroxide was added with stirring with a mechanical stirrer, to achieve pH 10. Addition of 2.50 mL of epichlorohydrin thereto at an inner temperature of 25°C and stirring of the resulting mixture caused solidification of the mixture 23 minutes later. After stopping the stirring, the mixture was left to stand at 25°C for 18 hours. To this mixture, 75 mL of 2-propaol was added, and the gel was crushed, followed by filtration of the resultant. The operation wherein the solids were added to 340 mL of water and the resulting mixture was stirred for 1 hour, followed by filtration thereof was repeated 3 times. The resultant was added to 600 mL of 2-propanol and the resulting mixture was stirred for 1 hour, followed by filtration thereof. The resultant was dried in a vacuum oven at 30°C for 37 hours to obtain 25.7 g of white solids. These were freeze-crushed to obtain sevelamer hydrochloride.

To confirm that sevelamer hydrochloride having a desired phosphate adsorption capability was synthesized, the phosphate adsorption capability of the sevelamer hydrochloride in the presence of phosphate alone was evaluated according to the *in vitro* assay method described in the above-described Patent Literature 1. More concretely, sodium carbonate, sodium chloride and disodium hydrogen phosphate dodecahydrate were used, and a solution wherein their

concentrations were 30 mM, 80 mM and 12 mM, respectively, was prepared, followed by addition thereto 1 M hydrochloric acid to adjust the pH to 7 to obtain a test solution. Subsequently, 20 mg of the sevelamer hydrochloride was placed in an Erlenmeyer flask and 10 mL of the above-described test solution was added thereto, followed by stirring at 37°C for 3 hours in a water bath. The pH became 8 to 9 after the stirring, and the pH of the resulting suspension was adjusted to 7 by 1 M hydrochloric acid. A small aliquot of this suspension was taken and sevelamer hydrochloride was removed therefrom by a centrifuge (refrigerated centrifuge 5417R manufactured by Eppendorf, angle rotor FA-45-24-11), followed by measuring (n=3) the amount of phosphate which was not adsorbed to the sevelamer hydrochloride using an inorganic phosphate measurement reagent (manufactured by Wako Pure Chemical Industries; Phosphor C (registered trademark)), to determine from the measured value the amount of the phosphate ions adsorbed to the sevelamer hydrochloride, that is, the phosphate adsorption capacity (a calibration curve was prepared within the range of the inorganic phosphate concentration of 0 to 386 mg/mL; the spectrophotometer was Spectra Max Plus manufactured by Molecular Devices). As a result, it was revealed that the phosphate adsorption capacity in the presence of phosphate alone was $3.4 \pm 0.5$ mmol/g, which is equivalent to the phosphate adsorption capacity described in Patent Literature 1 (3.1 mmol/g).

Subsequently, *in vitro* assays of the degree of swelling and the phosphate adsorption capability of the obtained sevelamer hydrochloride were carried out by the method described below.

[Measurement of Degree of Swelling of Sample]

In 50 mL of distilled water, 200 mg of a sample to be measured which was dried under reduced pressure at 40°C for not less than 16 hours was soaked for not less than 24 hours, and the sample was filtered under reduced pressure using a membrane filter having a diameter of 47 mm and pore size of 0.45 μm (Omnipore, manufactured by Millipore) to separate the solid component, whose weight was then divided by the dry weight (200 mg).

[Measurement of Phosphate Adsorption Capacity and Bile Acid Adsorption Capacity]

After stirring 10 mg of a sample to be measured in 1 mL of 50 mM hydrochloric acid at 37°C for 1 hour, 9 mL of a mixed solution of 11.1 mM each of disodium hydrogen phosphate dodecahydrate and an aqueous sodium glycocholate solution was added thereto. After stirring the resulting mixture at 37°C for additional 1 hour, the sample to be measured was removed therefrom by a centrifuge (refrigerated centrifuge 5417R manufactured by Eppendorf, angle rotor FA-45-24-11; 15000 rpm, 25°C, 15 minutes), followed by measuring (n=3) the amounts of phosphate and glycocholate which were not adsorbed to the sample to be measured using an inorganic phosphate measurement reagent (manufactured by Wako Pure Chemical Industries; Phosphor C (registered trademark)) and a bile acid measurement reagent (manufactured by Wako Pure Chemical Industries; Total Bile Acids (registered trademark)), respectively, to determine from the measured values the amount of the phosphate ions adsorbed to the sample to be measured, that is, the phosphate adsorption capacity (a calibration curve was prepared within the range of the inorganic phosphate concentration of 0 to 386 mg/mL; the spectrophotometer was Spectra Max Plus manufactured by Molecular Devices). The phosphate selectivity was indicated as the value calculated by dividing the phosphate adsorption capacity by the bile acid adsorption capacity.

The assay results of sevelamer hydrochloride are shown in Table 1. The phosphate selectivity was as low as 1.2, and the degree of swelling was 6.2.

[Table 1]

| Assay Results of Sevelamer Hydrochloride | | | | |
|---|---|---|---|---|
| | Degree of swelling | Phosphate adsorption capacity (mmol/g) | Bile acid adsorption capacity (mmol/g) | Phosphate selectivity |
| Reference Example 6 | 6.2 | 2.70 | 2.30 | 1.2 |

(Example 1)

Cross-linking Copolymerization of Allylammonium Dihydrogen Phosphate under Various Conditions, Alkali Treatment and Hydrochloride Formation Thereof

Polymerization to obtain the cross-linked polyallylamine or the acid addition salt thereof of the present invention

was carried out under various conditions. Table 2 shows the amount of the solvent, the amount of the polymerization initiator, the polymerization temperature and the polymerization time in each polymerization condition. Polymerization and hydrochloride formation of the obtained cross-linked polyallylamine phosphate under each condition were carried out by the following procedure. To a flask, 6.20 g of allyl ammonium dihydrogen phosphate, 2.80 g of *N,N'*-diallyl-1,3-diaminopropane bis(dihydrogen phosphate) and a predetermined amount of a solvent (water) were placed, and the resulting mixture was heated in an oil bath at 50°C to be dissolved. After replacing the atmosphere in the system with argon 3 times, a predetermined amount of a polymerization initiator (2,2'-azobis(2-amidinopropane) dihydrochloride) was added to the above solution, and the resulting mixture was heated at a predetermined polymerization temperature under stirring for a predetermined polymerization time period. The obtained solids were crushed and recovered by filtration, and sufficiently washed with water and then ethanol. The solids were then dried under reduced pressure at 60°C to obtain white powder of a cross-linked polyallylamine phosphate. The thus obtained cross-linked polyallylamine phosphate was dispersed in water (2.5 mL with respect to 1 g of the polymer), and 20% sodium hydroxide solution (4 mL with respect to 1 g of the polymer) was added thereto with stirring. After 30 minutes of stirring of the resulting mixture, solids were recovered by filtration. The obtained solids were washed with water until the filtrate became neutral and then washed with ethanol. This was followed by drying under reduced pressure at 60°C to obtain a free-form cross-linked polyallylamine. To a flask, 0.40 g of the obtained free-form cross-linked polyallylamine and 8 mL of water were placed, and 4 mL of concentrated hydrochloric acid was added thereto. After stirring the resulting mixture, solids were recovered by filtration. The obtained solids were washed with water until the filtrate becomes neutral and then washed with ethanol sufficiently. This was followed by drying under reduced pressure at 60°C to obtain a cross-linked polyallylamine hydrochloride.

Each of the obtained polyallylamine hydrochloride was assayed in the same manner as in Reference Example 6. The results are shown in Table 2. Comparison between the cases of polymerization at 55°C for 15 hours (Reference Example 7, Example 1-2, Example 1-4) and the cases of polymerization at 45°C for 61 hours (Example 1-1, Example 1-3, Example 1-5) showed that the cases of polymerization at 45°C for 61 hours exhibited somewhat lower phosphate adsorption capacities, but higher phosphate selectivities and lower degrees of swelling. When the amount of the initiator was doubled, the phosphate adsorption capacity somewhat decreased but the degree of swelling was found to decrease (Example 1-2, Example 1-3). Even in cases where the amount of the water used as the solvent was reduced by half, the degree of swelling was found to decrease (Example 1-4, Example 1-5). By optimizing the reaction temperature, the reaction time, the amount of the solvent and the amount of the initiator to be added, a cross-linked polyallylamine hydrochloride having a high phosphate adsorption capacity and a low degree of swelling was obtained.

[Table 2]

| Assay Results of Cross-linked Polyallylamine Hydrochloride Synthesized under Various Conditions | | | | | | | | | |
|---|---|---|---|---|---|---|---|---|---|
| | Polymerization initiator | | water (mL) | Temperature (°C) | Time (h) | Degree of swelling | Phosphate adsorption capacity (mmol/g) | Bile acid adsorption capacity (mmol/g) | Phosphate selectivity |
| | (g) | (mol%) a) | | | | | | | |
| Reference Example 7 | 0.11 | 0.83 | 8 | 55 | 15 | 6.0 | 3.70 | 1.39 | 2.7 |
| Example 1-1 | 0.11 | 0.83 | 8 | 45 | 61 | 4.0 | 3.11 | 0.75 | 4.1 |
| Example 1-2 | 0.22 | 1.7 | 8 | 55 | 15 | 4.0 | 3.03 | 1.03 | 2.9 |
| Example 1-3 | 0.22 | 1.7 | 8 | 45 | 61 | 3.9 | 2.91 | 0.64 | 4.5 |
| Example 1-4 | 0.11 | 0.83 | 4 | 55 | 15 | 3.8 | 2.80 | 1.25 | 2.2 |
| Example 1-5 | 0.11 | 0.83 | 4 | 45 | 61 | 3.0 | 2.73 | 0.76 | 3.6 |
| a) mol% with respect to the total number of moles of the monomers and the cross-linking agent | | | | | | | | | |

(Example 2)

Cross-linking Copolymerization of Allylammonium Dihydrogen Phosphate with Various Amounts of Cross-linking Agent to Be Added and Alkali treatment and Hydrochloride Formation Thereof

Polymerization to obtain the cross-linked polyallylamine or the acid addition salt thereof of the present invention was carried out with various amounts of the added cross-linking agent as described in Table 3. Polymerization and hydrochloride formation of the obtained cross-linked polyallylamine phosphate under each condition were carried out by the following procedure. To a flask, 6.20 g of allylammonium dihydrogen phosphate, a predetermined amount of the cross-linking agent (*N,N'*-diallyl-1,3-diaminopropane bis(dihydrogen phosphate)) and 4 mL of water were placed, and the resulting mixture was heated in an oil bath at 50°C to be dissolved. After replacing the atmosphere in the system with argon 3 times, 0.11 g of 2,2'-azobis(2-amidinopropane) dihydrochloride was added to the above solution, and the resulting mixture was heated at an inner temperature of 45 to 47°C for 64 hours with stirring. Several hours after the addition of 2,2'-azobis(2-amidinopropane) dihydrochloride, the reaction system became solidified, and the stirring was stopped. The obtained solids were crushed and recovered by filtration, and sufficiently washed with water and then ethanol. The solids were then dried under reduced pressure at room temperature to obtain white powder of a cross-linked polyallylamine phosphate. The thus obtained cross-linked polyallylamine phosphate was dispersed in water (5 mL with respect to 1 g of the polymer), and 20% sodium hydroxide solution (4 mL with respect to 1 g of the polymer) was added thereto with stirring. After 1 hour of stirring of the resulting mixture at room temperature, solids were recovered by filtration and washed with water until the filtrate became neutral. The solids were then stirred in water overnight and recovered by filtration, followed by washing thereof with ethanol. After drying under reduced pressure, a free-form cross-linked polyallylamine was obtained. To a flask, 0.40 g of the obtained free-form cross-linked polyallylamine and 8 mL of water were placed, and 2 mL of concentrated hydrochloric acid was added thereto. After stirring the resulting mixture for 30 minutes, solids were recovered by filtration. The obtained solids were washed with water until the filtrate became neutral and then washed with ethanol. This was followed by drying under reduced pressure at room temperature to obtain a cross-linked polyallylamine hydrochloride.

Each obtained polyallylamine hydrochloride was assayed in the same manner as in Reference Example 6. The results are shown in Table 3. With the amount of the added cross-linking agent of 2 mol%, the degree of swelling was high, so that the object of the present invention could not be achieved, but, as the amount of the additive increased, the degree of swelling decreased and the phosphate selectivity was improved at the same time. With the amount of the added cross-linking agent of not less than 5 mol%, the degree of swelling was less than 5.0. However, the degree of swelling was found not to have decreased even by increasing the amount of the added cross-linking agent from 20 mol% to 30 mol%, so that it was thought that the cross-linking did not proceed efficiently.

[Table 3]

Assay Results of Cross-linked Polyallylamine Hydrochloride Synthesized with Various Amounts of Added Cross-linking Agent

| | Amount of added cross-linking agent (mol%) | Degree of swelling | Phosphate adsorption capacity (mmol/g) | Bile acid adsorption capacity (mmol/g) | Phosphate selectivity |
|---|---|---|---|---|---|
| Reference Example 8 | 2 | 7.9 | 3.06 | 2.66 | 1.2 |
| Example 2-1 | 5 | 4.3 | 3.42 | 2.15 | 1.6 |
| Example 2-2 | 10 | 3.5 | 3.52 | 1.16 | 3.0 |
| Example 2-3 | 15 | 3.2 | 3.86 | 1.29 | 3.0 |
| Example 2-4 | 20 | 3.1 | 3.21 | 1.17 | 2.7 |
| Reference Example 9 | 30 | 3.2 | 2.29 | 1.10 | 2.1 |

(Example 3)

Cross-linking Copolymerization of Allylammonium Dihydrogen Phosphate with Various Amounts of Cross-linking Agent Added, Alkali treatment and Hydrochloride Formation Thereof (with Usage of Cross-linking Agent of Product Purified by Recrystallization)

Polymerization to obtain the cross-linked polyallylamine or the acid addition salt thereof of the present invention was carried out with various amounts of the added cross-linking agent as described in Table 4. Polymerization and hydrochloride salification of the obtained cross-linked polyallylamine phosphate under each condition were carried out in the same manner as in Example 2 except that the amounts of allylammonium dihydrogen phosphate which is the monomer, water which is the solvent, and 2,2'-azobis(2-amidinopropane) dihydrochloride which is the initiator were changed as appropriate according to the description in Table 4 and that *N,N'*-diallyl-1,3-diaminopropane bis(dihydrogen phosphate) purified by recrystallization was used as the predetermined amount of the cross-linking agent.

Each obtained polyallylamine hydrochloride was assayed in the same manner as in Reference Example 6. The results are shown in Table 4. With the amount of the added cross-linking agent of 2 mol%, the degree of swelling was high, so that the object of the present invention could not be achieved, but, as the amount of the additive increased, the degree of swelling decreased and the phosphate selectivity was improved at the same time. With the amount of the added cross-linking agent of not less than 5 mol%, the degree of swelling was less than 5.0. The degree of swelling was found not to have decreased even by increasing the amount of the added cross-linking agent from 20 mol% to 30 mol%.

[Table 4]

Assay Results of Cross-linked Polyallylamine Hydrochloride Synthesized with Various Amounts of Added Cross-linking Agent

| | Amount of added cross-linking agent (mol%) | Monomer (g) | Cross-linking agent (g) | water (mL) | Initiator | | degree of swelling | Phosphate adsorption capacity (mmol/g) | Bile acid adsorption capacity (mmol/g) | Phosphate selectivity |
|---|---|---|---|---|---|---|---|---|---|---|
| | | | | | (g) | (mol%) a) | | | | |
| Reference Example 10 | 2 | 9.31 | 0.42 | 6.0 | 0.16 | 0.98 | 8.0 | 3.26 | 3.10 | 1.1 |
| Example 3-1 | 5 | 9.31 | 1.05 | 6.0 | 0.16 | 0.95 | 4.2 | 3.53 | 2.31 | 1.5 |
| Example 3-2 | 10 | 8.53 | 1.93 | 5.5 | 0.16 | 0.91 | 3.6 | 3.71 | 1.36 | 2.7 |
| Example 3-3 | 15 | 7.76 | 2.63 | 5.0 | 0.16 | 0.87 | 4.0 | 4.08 | 1.59 | 2.6 |
| Example 3-4 | 20 | 7.76 | 3.50 | 5.0 | 0.16 | 0.83 | 3.2 | 4.23 | 1.57 | 2.7 |
| Example 3-5 | 30 | 6.20 | 4.20 | 4.0 | 0.16 | 0.77 | 3.3 | 3.70 | 1.76 | 2.1 |

a) mol% with respect to the total number of moles of the monomers and the cross-linking agent

(Comparative Example 1)

Cross-linking Copolymerization of Allylammonium Dihydrogen Phosphate Using Various Cross-linking Agents, Alkali treatment and Hydrochloride Formation Thereof

Polymerization to obtain the cross-linked polyallylamine or the acid addition salt thereof of the present invention was carried out with various cross-linking agents and various amounts of the added cross-linking agents as described in Table 5. Polymerization and hydrochloride formation of the obtained cross-linked polyallylamine phosphate under each condition were carried out in the same manner as in Example 2 except that the cross-linking agent and the amount of the added cross-linking agent were changed as appropriate according to the description in Table 5.

Each of the obtained polyallylamine hydrochloride was assayed in the same manner as in Reference Example 6. The results are shown in Table 5. The data from Example 3-1 wherein *N,N'*-diallyl-1,3-diaminopropane bis(dihydrogen phosphate) was used as the cross-linking agent in an amount of 5% are also shown. In cases where the diphosphate of each of *N,N'*-diallyl-1,2-diaminoethane, *N,N'*-diallyl-1,4-diaminobutane and *N,N'*-diallyl-1,5-diaminopentane was used as the cross-linking agent, the degree of swelling was high with any amount of the added cross-linking agent of 5 mol% to 30 mol%, so that the object of the present invention could not be achieved. Thus, it is necessary to use an acid addition salt of *N,N'*-diallyl-1,3-diaminopropane as the cross-linking agent.

[Table 5]

| Assay Results of Cross-linked Polyallylamine Hydrochloride Synthesized Using Various Cross-linking Agents | | | | | | |
|---|---|---|---|---|---|---|
| | Cross-linking agent (See Note) | Amount of added cross-linking agent (mol%) | degree of swelling | Phosphate adsorption capacity (mmol/g) | Bile acid adsorption capacity (mmol/g) | Phosphate selectivity |
| Comparative Example 1-1 | C2 | 2 | 9.2 | 2.85 | 3.35 | 0.9 |
| Comparative Example 1-2 | C2 | 5 | 5.9 | 3.10 | 2.13 | 1.5 |
| Comparative Example 1-3 | C2 | 10 | 5.0 | 2.98 | 1.74 | 1.7 |
| Comparative Example 1-4 | C2 | 15 | 5.1 | 3.12 | 2.59 | 1.2 |
| Comparative Example 1-5 | C2 | 20 | 4.9 | 2.96 | 2.19 | 1.4 |
| Comparative Example 1-6 | C2 | 30 | 5.9 | 2.63 | 2.30 | 1.1 |
| Comparative Example 1-7 | C4 | 2 | 7.5 | 3.00 | 3.18 | 0.9 |
| Comparative Example 1-8 | C4 | 5 | 5.0 | 3.39 | 2.75 | 1.2 |
| Comparative Example 1-9 | C4 | 10 | 5.6 | 3.29 | 2.70 | 1.2 |
| Comparative Example 1-10 | C4 | 15 | 5.1 | 3.19 | 3.71 | 0.9 |
| Comparative Example 1-11 | C4 | 20 | 5.2 | 2.95 | 3.45 | 0.9 |
| Comparative Example 1-12 | C4 | 30 | 5.7 | 2.75 | 3.60 | 0.8 |
| Comparative Example 1-13 | C5 | 2 | 8.2 | 2.66 | 2.94 | 0.9 |

(continued)

| Assay Results of Cross-linked Polyallylamine Hydrochloride Synthesized Using Various Cross-linking Agents | | | | | | |
|---|---|---|---|---|---|---|
| | Cross-linking agent (See Note) | Amount of added cross-linking agent (mol%) | degree of swelling | Phosphate adsorption capacity (mmol/g) | Bile acid adsorption capacity (mmol/g) | Phosphate selectivity |
| Comparative Example 1-14 | C5 | 5 | 5.6 | 3.02 | 2.98 | 1.0 |
| Comparative Example 1-15 | C5 | 10 | 7.0 | 2.65 | 3.18 | 0.8 |
| Comparative Example 1-16 | C5 | 15 | 6.3 | 1.81 | 2.71 | 0.7 |
| Comparative Example 1-17 | C5 | 20 | 7.6 | 1.79 | 2.61 | 0.7 |
| Comparative Example 1-18 | C5 | 30 | 8.1 | 1.76 | 3.00 | 0.6 |
| Example 3-1 | C3 | 5 | 4.2 | 3.53 | 2.31 | 1.5 |
| (Note) C2: *N,N'*-diallyl-1,2-diaminoethane bis(dihydrogen phosphate), C3: *N,N'*-diallyl-1,3-diaminopropane bis (dihydrogen phosphate), C4: *N,N'*-diallyl-1,4-diaminobutane bis(dihydrogen phosphate), C5: *N,N'*-diallyl-1,5-diaminopentane bis(dihydrogen phosphate) | | | | | | |

(Example 4)

Study to Decrease Amount of Hydrochrolic Acid

A cross-linked polyallylamine hydrochloride releases hydrochloric acid upon adsorption of phosphate thereto. Since excessive release of hydrochloric acid may cause hyperchloremic acidosis, the degree of hydrochloride formation is preferably as low as possible. Therefore, the free-form cross-linked polyallylamine obtained under the conditions of Example 2-4 shown in Table 3 (the cross-linking agent was used in an amount of 20 mol%) was used to confirm the effect obtained in cases where a part of the amino groups were converted to hydrochloride (a cross-linked polyallylamine wherein two thirds of the amino groups were subjected to hydrochloride formation is hereinafter described as a "cross-linked polyallylamine 2/3 hydrochloride").

(1) Complete hydrochloride (Example 4-1)

In 60 mL of water, 3.00 g of a free-form cross-linked polyallylamine obtained under the conditions of Example 2-4 was dispersed, and 30 mL of concentrated hydrochloric acid was added thereto at room temperature. After stirring the resulting mixture for 1.5 hours, solids were recovered by filtration. The obtained solids were washed with water until the filtrate became neutral and then washed with ethanol sufficiently. This was followed by drying under reduced pressure at room temperature to obtain 4.84 g of a cross-linked polyallylamine hydrochloride.

(2) 2/3 hydrochloride (Example 4-2)

In 4.0 mL of water, 0.35 g of a free-form cross-linked polyallylamine obtained under the conditions of Example 2-4 was dispersed, and 3.7 mL of 1M hydrochloric acid was added thereto at room temperature. After stirring the resulting mixture for 1 hour, solids were recovered by filtration. The recovered solids were washed with water and then washed sufficiently with ethanol. This was followed by drying under reduced pressure at room temperature to obtain 0.53 g of a cross-linked polyallylamine 2/3 hydrochloride.

The obtained polyallylamine hydrochloride was assayed in the same manner as in Reference Example 6. The results are shown in Table 6. The degrees of swelling, phosphate adsorption capacities and phosphate selectivities were similar irrespective of the degree of hydrochloride formation, and it was revealed that it is possible to reduce the number of equivalence of hydrochloric acid used for the salt formation to two thirds. Although not shown in the Table, the phosphate adsorption capacity could be increased to 4.79 mmol/g in a case where the diameter of the polymer particle

could be reduced by the operation of stirring the free-form cross-linked polyallylamine in water overnight upon alkali treatment (the bile acid adsorption capacity was 1.21 mmol/g, the phosphate selectivity was 4.0, and the degree of swelling was 4.0).

[Table 6]

| Assay Results of Cross-linked Polyallylamine 2/3 Hydrochloride Synthesized | | | | | |
|---|---|---|---|---|---|
| | Equivalence of hydrochloric acid | Degree of swelling | Phosphate adsorption capacity (mmol/g) | Bile acid adsorption capacity (mmol/g) | Phosphate selectivity |
| Example 4-1 | 1 | 3.3 | 3.61 | 1.42 | 2.5 |
| Example 4-2 | 2/3 | 3.3 | 3.73 | 1.16 | 3.2 |

(Comparative Example 2)

Comparison with Cross-linked Polymer of Allylammonium Chloride

In place of allylammonium dihydrogen phosphate which is the monomer of the present invention, allylammonium chloride which is the hydrochloride of allylamine was used for the polymerization. Polymerization and hydrochloride formation of the obtained cross-linked polyallylamine phosphate were carried out in the same manner as in Example 2 except that 3.74 g (40 mmol) of allylammonium chloride was used as the monomer and 1.82 g (8 mmol) of *N,N'*-diallyl-1,3-diaminopropane dihydrochloride was used as the cross-linking agent. The obtained cross-linked polyallylamine hydrochloride was assayed in the same manner as in Reference Example 6. The results are shown in Table 7. Compared to the cross-linked polyallylamine hydrochloride which differs only in usage of allylammonium dihydrogen phosphate as the monomer (Example 2-4), the cross-linked polyallylamine hydrochloride obtained using allylammonium chloride as the monomer showed a lower phosphate selectivity and a lower phosphate adsorption capacity. Thus, it was revealed that it is necessary to use allylammonium dihydrogen phosphate which is the phosphate of allylamine as the monomer of the present invention.

[Table 7]

| Assay Results of Polymers Obtained by Polymerization with Hydrochloride | | | | |
|---|---|---|---|---|
| | Degree of swelling | Phosphate adsorption capacity (mmol/g) | Bile acid adsorption capacity (mmol/g) | Phosphate selectivity |
| Comparative Example 2 | 3.5 | 2.56 | 1.50 | 1.7 |
| Example 2-4 | 3.1 | 3.21 | 1.17 | 2.7 |

(Reference Example 11)

Application of Molecular Imprinting Using Potassium Dihydrogen Phosphate (Part 1)

To confirm whether or not the method of phosphate imprinting described in the above-described Patent Literature 4 (US 2005/0276781 A1) is applicable to the allylamine used in the present invention, we attempted synthesis of a phosphate-imprinted polymer using allylamine according to the method described in Patent Literature 4. To a flask, 3.0 mL (40 mmol) of allylamine and 1.23 g (8 mmol) of *N,N'*-diallyl-1,3-diaminopropane were placed, and 2 mL of 2-propanol or water was added thereto. To the resulting mixture, 0.71 g (5.2 mmol) of potassium dihydrogen phosphate was added and stirred for 3 hours at room temperature. To the resulting mixture, 0.108 g (0.4 mmol) of 2,2'-azobis(2-amidinopropane) dihydrochloride was added, and the atmosphere in the system was replaced with argon 3 times, followed by heating the mixture at 50°C for 60 hours with stirring. After cooling the mixture to room temperature, water was added thereto only to form a homogeneous solution in either case, so that a water-insoluble polymer could not be obtained. From this result,

it was revealed that it is impossible to polymerize allylamine in the presence of potassium dihydrogen phosphate, and that the method of phosphate imprinting described in Patent Literature 4 is not applicable to the allylamine used in the present invention.

(Reference Example 12)

Application of Molecular Imprinting using Potassium Dihydrogen Phosphate (Part 2)

The potassium dihydrogen phosphate used for the molecular imprinting in the above-described Patent Literature 4 was used in an amount of 1 equivalent with respect to the amino groups contained in allylamine and the cross-linking agent to convert all the amino groups to hydrochlorides, thereby polymerization was attempted. To a flask, 3.0 mL (40 mmol) of allylamine and 1.23 g (8 mmol) of *N,N'*-diallyl-1,3-diaminopropane were placed and 4 mL of water was added thereto. To the resulting mixture, 7.62 g (56 mmol) of potassium dihydrogen phosphate was added, thereby precipitating white solids, and stirring became difficult. The resultant was left to stand at room temperature for 3 hours, and 4 mL of water was added thereto, followed by heating the mixture at 50°C with stirring to dissolve the solids. To the resulting solution, 0.108 g (0.4 mmol) of 2,2'-azobis(2-amidinopropane) dihydrochloride was added, and the atmosphere in the system was replaced with argon 3 times, followed by heating the mixture at 50°C for 110 hours with stirring. No generation of solids was observed. After cooling the mixture to room temperature, water was added thereto only to form a homogeneous solution, so that a water-insoluble polymer could not be obtained. According to this result, potassium allylammonium hydrogen phosphate obtained by mixing allylamine and potassium dihydrogen phosphate is inappropriate for obtaining a polymer by polymerizing the phosphate of allylamine. It was revealed that, to solve the problem which is to be solved by the present invention, it is indispensable to use allylammonium dihydrogen phosphate obtained by mixing allylamine and phosphoric acid at a ratio of 1:1, and that a desired cross-linked polyallylamine can be obtained only in such a case.

(Example 5)

Urinary Phosphate Excretion Test in Normal Rat

(Synthesis of Sample for Test and *in Vitro* Assay)

To a flask, 46.5 g (300 mmol) of allylammonium dihydrogen phosphate, 21.0 g (60 mmol) of *N,N'*-diallyl-1,3-diaminopropane bis(dihydrogen phosphate) and 30.0 mL of water were placed and the atmosphere in the system was replaced with argon 3 times, followed by heating the mixture in an oil bath at 50°C to dissolve it. The atmosphere in the system was replaced once with argon, and the mixture was bubbled with argon at room temperature for 30 minutes. To this mixture, 0.814 g (3 mmol) of 2,2'-azobis(2-amidinopropane) dihydrochloride was added with stirring, and the resulting mixture was heated at an inner temperature of 47°C for 65.5 hours. Several hours after the beginning of the heating, the mixture became solidified, and stirring was stopped. The obtained solids were crushed and recovered by filtration, and sufficiently washed with water and then ethanol. The solids were then dried under reduced pressure at 60°C to obtain 51.7 g of white powder of a cross-linked polyallylamine phosphate. In 75 mL of water, 51.0 g of the obtained cross-linked polyallylamine phosphate was dispersed, and 200 g of 20% aqueous sodium hydroxide solution was added thereto with stirring. Since salt precipitated, 75 mL of water was added to the mixture, and the resulting mixture was stirred at room temperature for 30 minutes, followed by recovery of solids by filtration and washing thereof with water. The solids were transferred to an Erlenmeyer flask, and 500 mL of water was added thereto, followed by 14 hours of stirring at room temperature. The solids were recovered by filtration, and washed with water and then ethanol. The solids were then dried at 60°C under reduced pressure to obtain 17.5 g of a free-form cross-linked polyallylamine. To a round bottom flask, 4.50 g of the obtained free-form cross-linked polyallylamine and 50 mL of water were placed, and 47.8 mL of 1M hydrochloric acid was added to the resulting mixture. After stirring the mixture at room temperature for 1 hour, solids were recovered by filtration. The recovered solids were washed with water and then ethanol. This was followed by drying under reduced pressure at 50°C to obtain 6.51 g of a cross-linked polyallylamine 2/3 hydrochloride. By freeze-crushing 5.00 g of the obtained cross-linked polyallylamine 2/3 hydrochloride, 4.94 g of a freeze-crushed product of the cross-linked polyallylamine 2/3 hydrochloride was obtained. The obtained freeze-crushed product of the cross-linked polyallylamine 2/3 hydrochloride was assayed in the same manner as in Reference Example 6. The results are shown in Table 8. As Comparative Example 3, the assay results of sevelamer hydrochloride in Reference Example 6 are shown.

[Table 8]

| Assay Results of Polymers for *in Vivo* Test | | | | |
|---|---|---|---|---|
| | Degree of swelling | Phosphate adsorption capacity (mmol/g) | Bile acid adsorption capacity (mmol/g) | Phosphate selectivity |
| Example 5 | 3.6 | 3.82 | 1.09 | 3.5 |
| Comparative Example 3 (Reference Example 6) | 6.2 | 2.70 | 2.30 | 1.2 |

(*In Vivo* Test)

SD rats (SPF, male, 6 weeks old, JAPAN SLC) were separately kept under a time-restricted feeding schedule of 8 hours/day with a feeding amount of 10 g/day. After about 1 week of habituation, the polymer in Example 5 or sevelamer hydrochloride (Comparative Example 3 (Reference Example 6)) was mixed with a feed in an amount of 0.3% by weight or 1% by weight, and the rats were fed with the mixture for 3 days. Rats in the control group were fed with only the feed. During the 3 days of feeding, urine was collected for 24 hours/day and the daily urinary phosphate excretion amounts were measured to obtain the total sum of the amount during the 3 days. A decrease in the urinary phosphate excretion amount indicates the phosphate adsorption effect by the cross-linked polyallylamine hydrochloride or sevelamer hydrochloride.

The results are shown in Fig. 1. The cross-linked polyallylamine hydrochloride in Example 5 showed a dose-dependent and significant decrease in the phosphate excretion amount compared to the control group, and sevelamer hydrochloride also showed the same level of the urinary phosphate excretion-reducing effect. Therefore, it was shown that, in spite of the fact that the cross-linked polyallylamine hydrochloride in Example 5 has a lower degree of swelling than sevelamer hydrochloride, it has the same level of the phosphate adsorption effect as sevelamer hydrochloride.

(Comparative Example 4)

Synthesis of Polymer Using *N,N'*-diallyl-1,4-diaminobutane Bis(dihydrogen phosphate) as Cross-linking Agent

The free-form cross-linked polyallylamine obtained during the process of synthesis of the cross-linked polyallylamine hydrochloride in Comparative Example 1-8 (*N,N'*-diallyl-1,4-diaminobutane bis(dihydrogen phosphate) was used as the cross-linking agent in an amount of 5 mol% with respect to the monomers) was used for the synthesis. To a round bottom flask, 3.30 g of the free-form cross-linked polyallylamine and 37 mL of water were placed and 37.4 mL of 1M hydrochloric acid was added thereto with stirring. After stirring the resulting mixture for 30 minutes at room temperature, solids were recovered by filtration. The recovered solids were washed with water and then ethanol. This was followed by drying under reduced pressure at 50°C to obtain 4.60 g of a cross-linked polyallylamine 2/3 hydrochloride.

(Example 6)

Urinary Phosphate Excretion Test in Normal Rats (Comparison among Cross-linking Agents)

(Synthesis of Sample for Test)

The free-form cross-linked polyallylamine obtained during the process of synthesis of the cross-linked polyallylamine hydrochloride in Example 3-1 (*N,N'*-diallyl-1,3-diaminopropane bis(dihydrogen phosphate) was used as the cross-linking agent in an amount of 5 mol% with respect to the monomers) was used for the synthesis. To a round bottom flask, 2.70 g of the free-form cross-linked polyallylamine and 30 mL of water were placed, and 30.6 mL of 1M hydrochloric acid was added thereto with stirring. After stirring the resulting mixture for 1 hour at room temperature, solids were recovered by filtration. The recovered solids were washed with water and then ethanol. This was followed by drying under reduced pressure at 50°C to obtain 3.89 g of a cross-linked polyallylamine 2/3 hydrochloride.

(*In Vivo* Test)

SD rats (SPF, male, 6 weeks old, JAPAN SLC) were separately kept under a time-restricted feeding schedule of 8 hours/day with a feeding amount of 10 g/day. After about 1 week of habituation, the cross-linked polyallylamine hydrochloride in Example 6, the cross-linked polyallylamine hydrochloride in Comparative Example 4, or sevelamer hydrochloride (Comparative Example 3 (Reference Example 6)) was mixed with a feed in an amount of 1% by weight, and the rats were fed with the mixture for 3 days. Rats in the control group were fed with only the feed. During the 3 days of feeding, urine was collected for 24 hours/day and the daily urinary phosphate excretion amounts were measured to obtain the total sum of the amount during the 3 days. A decrease in the urinary phosphate excretion amount indicates the phosphate adsorption effect by the cross-linked polyallylamine hydrochloride or sevelamer hydrochloride.

The results are shown in Fig. 2. The cross-linked polyallylamine hydrochloride in Example 6 showed a significant decrease in the phosphate excretion amount compared to the control, and showed a urinary phosphate excretion-reducing effect higher than that of sevelamer hydrochloride. On the other hand, while the cross-linked polyallylamine hydrochloride in Comparative Example 4 showed a significant decrease in the phosphate excretion amount compared to the control, its effect was weaker than that of sevelamer hydrochloride and the cross-linked polyallylamine hydrochloride in Example 6. The cross-linked polyallylamine hydrochloride in Example 6 was shown to have a phosphate adsorption effect not less than that of sevelamer hydrochloride. On the other hand, the cross-linked polyallylamine hydrochloride in Comparative Example 4 using as the cross-linking agent *N,N'*-diallyl-1,4-diaminobutane diphosphate was shown to have a phosphate adsorption effect lower than that of the cross-linked polyallylamine hydrochloride in Example 6 and sevelamer hydrochloride.

(Example 7)

Synthesis of Free-form Cross-linked Polyallylamine and Cross-linked Polyallylamine 2/3 Hydrochloride for Surface Cross-linking Study and *in Vitro* Assay of Cross-linked Polyallylamine 2/3 Hydrochloride

To a flask, 12.4 g (80 mmol) of allylammonium dihydrogen phosphate, 5.60 g (16 mmol) of *N,N'*-diallyl-1,3-diaminopropane bis(dihydrogen phosphate) and 8.0 mL of water were placed, and the atmosphere in the system was replaced with argon 3 times, followed by heating the mixture in an oil bath at 50°C to dissolve it. The mixture was bubbled with argon for 30 minutes. To this mixture, 0.814 g (3 mmol) of 2,2'-azobis(2-amidinopropane) dihydrochloride was added at an inner temperature of 50°C with stirring, and the resulting mixture was heated at an inner temperature of 49 to 51°C for 64 hours. Several hours after the beginning of the heating, the mixture became solidified, and stirring was stopped. The obtained solids were crushed and recovered by filtration, and washed with water and then ethanol. The solids were then dried under reduced pressure at room temperature to obtain 15.0 g of white powder of a cross-linked polyallylamine phosphate. In 90 mL of water, 15.0 g of the obtained cross-linked polyallylamine phosphate was dispersed, and 60 mL of 20% aqueous sodium hydroxide solution was added thereto with stirring. The resulting mixture was stirred at room temperature for 1 hour, and solids were recovered by filtration, followed by washing thereof with water until the filtrate becomes neutral. The solids were transferred to a beaker, and 100 mL of water was added thereto, followed by 16 hours of stirring at room temperature. The solids were recovered by filtration, and washed with water and then ethanol. This was followed by drying under reduced pressure at 50°C to obtain 5.12 g of a free-form cross-linked polyallylamine.

Into a test tube, 0.25 g of the obtained free-form cross-linked polyallylamine was weighed and dispersed in 5.0 mL of water. To the resulting mixture, 2.65 mL of 1 M hydrochloric acid was added with ice cooling, while stirring thereof with a magnetic stirrer. After 30 minutes of stirring of the resulting mixture, solids were recovered by filtration. The obtained solids were washed with water and then washed with ethanol sufficiently. This was followed by drying under reduced pressure at room temperature to obtain 0.38 g of a cross-linked polyallylamine 2/3 hydrochloride. The obtained cross-linked polyallylamine 2/3 hydrochloride was assayed in the same manner as in Reference Example 6. The results are shown in Table 9.

[Table 9]

| Assay Results of 2/3 Hydrochloride of the Polymer for Surface Cross-linking Study (Sample without Surface Cross-linking) | | | | |
|---|---|---|---|---|
| | Degree of swelling | Phosphate adsorption capacity (mmol/g) | Bile acid adsorption capacity (mmol/g) | Phosphate selectivity |
| Example 6 | 3.3 | 4.02 | 1.16 | 3.5 |

(Examples 8 to 12)

Study of Reaction Conditions for Surface Cross-linking of Free-form Cross-linked Polyallylamine

The reaction conditions were studied using as the surface cross-linking agent 2-hydroxyethyl acrylate. In 4.0 mL of a solvent shown in Table 10, 0.25 g of the free-form cross-linked polyallylamine obtained in Example 7 was dispersed, and the resulting mixture was stirred at a temperature shown in Table 10. In 2.0 mL of the same solvent, 9.2 mg of 2-hydroxyethyl acrylate (3.7% by weight with respect to the free-form cross-linked polyallylamine) was dissolved, and this solution was added to the above mixture, followed by stirring the resulting mixture for 1 hour. Solids were recovered by filtration and washed with the same solvent as the one used for the reaction, followed by drying under reduced pressure at room temperature. The obtained polymer was dispersed in 5.0 mL of water, and 2.65 mL of 1 M hydrochloric acid was added thereto with ice cooling and stirring. After 30 minutes of stirring of the mixture at room temperature, solids were recovered by filtration. The recovered solids were washed with water and then ethanol. The solids were dried under reduced pressure at room temperature to obtain a cross-linked polyallylamine 2/3 hydrochloride (surface cross-linked product). The obtained cross-linked polyallylamine 2/3 hydrochloride (surface cross-linked product) was assayed in the same manner as in Reference Example 6. The results are shown in Table 10. Compared to the case where surface cross-linking was not carried out (Example 7), the degree of swelling decreased with any of the solvents, although no large difference was observed for the phosphate adsorption capacity.

[Table 10]

| Study Results of Surface Cross-linking Conditions Using 2-Hydroxyethyl Acrylate | | | | | | |
|---|---|---|---|---|---|---|
| | Solvent | Temperature | Degree of swelling | Phosphate adsorption capacity (mmol/g) | Bile acid adsorption capacity (mmol/g) | Phosphate selectivity |
| Example 7 | - | - | 3.3 | 4.02 | 1.16 | 3.5 |
| Example 8 | ethanol | 50°C | 3.0 | 3.99 | 0.95 | 4.2 |
| Example 9 | ethyl acetate | 50°C | 3.0 | 3.79 | 1.04 | 3.6 |
| Example 10 | heptane | 50°C | 3.0 | 3.99 | 0.92 | 4.3 |
| Example 11 | ethanol | 30°C | 2.8 | 3.78 | 1.03 | 3.7 |
| Example 12 | heptane | 30°C | 3.0 | 3.77 | 1.06 | 3.6 |

(Examples 13 to 17)

Study of Amount of 2-Hydroxyethyl Acrylate to Be Added in Surface Cross-linking of Free-form Cross-linked Polyallylamine

2-Hydroxyethyl acrylate was used as the surface cross-linking agent, and its amount to be added was studied. In 4.0 mL of ethanol, 0.25 g of the free-form cross-linked polyallylamine obtained in Example 7 was dispersed, and the resulting mixture was stirred at 30°C. In 2.0 mL of ethanol, 2-hydroxyethyl acrylate in an amount shown in Table 11 was dissolved, and the resulting solution was added to the above mixture, followed by stirring the resulting mixture for 1 hour. Solids were recovered by filtration and washed with ethanol, followed by drying under reduced pressure at room temperature. The obtained polymer was dispersed in 5.0 mL of water, and 2.65 mL of 1 M hydrochloric acid was added thereto with ice cooling and stirring. After stirring thereof at room temperature for 30 minutes, solids were recovered by filtration. The recovered solids were washed with water and then ethanol. The solids were dried under reduced pressure at room temperature to obtain a cross-linked polyallylamine 2/3 hydrochloride (surface cross-linked product). The obtained cross-linked polyallylamine 2/3 hydrochloride (surface cross-linked product) was assayed in the same manner as in Reference Example 6. The results are shown in Table 11. The degree of swelling decreased depending on the amount of surface cross-linking agent added. Further, the phosphate adsorption capacity decreased depending on the amount of surface cross-linking agent added.

[Table 11]

| Results of Surface Cross-linking Using Various Amounts of Added 2-Hydroxyethyl Acrylate | | | | | |
|---|---|---|---|---|---|
| | Amount of 2-hydroxyethyl acrylate added (ratio with respect to the polymer) | Degree of swelling | Phosphate adsorption capacity (mmol/g) | Bile acid adsorption capacity (mmol/g) | Phosphate selectivity |
| Example 13 | 4.6 mg (1.8% by weight) | 3.1 | 4.02 | 1.16 | 3.5 |
| Example 14 | 9.2 mg (3.7% by weight) | 3.2 | 3.95 | 1.29 | 3.1 |
| Example 15 | 23.1 mg (9.2% by weight) | 2.8 | 3.73 | 1.08 | 3.5 |
| Example 16 | 46.2 mg (18% by weight) | 2.6 | 3.46 | 1.07 | 3.2 |
| Example 17 | 92.4 mg (37% by weight) | 2.4 | 2.91 | 0.98 | 3.0 |

(Examples 18 to 21)

Surface Cross-linking Using Methyl Acrylate or Epichlorohydrin

In 4.0 mL of ethanol or heptane, 0.25 g of the free-form cross-linked polyallylamine obtained in Example 7 was dispersed, and the resulting mixture was stirred at a temperature shown in Table 12. In 2.0 mL of the same solvent as the one used for dispersion of the above-described free-form cross-linked polyallylamine, methyl acrylate or epichlorohydrin was dissolved in an amount shown in Table 12, and the resulting solution was added to the above mixture, followed by stirring the resulting mixture for additional 1 hour. Solids were recovered by filtration and washed with the same solvent as the one used for the above-described cross-linking, followed by drying under reduced pressure at room temperature. The obtained polymer was dispersed in 5.0 mL of water, and 2.65 mL of 1 M hydrochloric acid was added thereto with ice cooling and stirring. After stirring thereof at room temperature for 30 minutes, solids were recovered by filtration. The recovered solids were washed with water and then ethanol sufficiently. The solids were dried under reduced pressure at room temperature to obtain a cross-linked polyallylamine 2/3 hydrochloride (surface cross-linked product). The obtained cross-linked polyallylamine 2/3 hydrochloride (surface cross-linked product) was assayed in the same manner as in Reference Example 6. The results are shown in Table 12. In all the cases, the degree of swelling decreased compared to that of the case where surface cross-linking was not carried out (Example 7).

[Table 12]

| Results of Surface Cross-linking Using Methyl Acrylate or Epichlorohydrin | | | | | | |
|---|---|---|---|---|---|---|
| | Surface cross-linking agent solvent/ temperature | Amount of surface cross-linking agent added (ratio with respect to the polymer) | Degree of swelling | Phosphate adsorption capacity (mmol/g) | Bile acid adsorption capacity (mmol/g) | Phosphate selectivity |
| Example 7 | - | - | 3.3 | 4.02 | 1.16 | 3.5 |
| Example 18 | methyl acrylate ethanol/30°C | 6.9 mg (2.8% by weight) | 2.9 | 3.93 | 0.93 | 4.2 |

(continued)

| Results of Surface Cross-linking Using Methyl Acrylate or Epichlorohydrin | | | | | | |
|---|---|---|---|---|---|---|
| | Surface cross-linking agent solvent/ temperature | Amount of surface cross-linking agent added (ratio with respect to the polymer) | Degree of swelling | Phosphate adsorption capacity (mmol/g) | Bile acid adsorption capacity (mmol/g) | Phosphate selectivity |
| Example 19 | methyl acrylate ethanol/30°C | 17.1 mg (6.8% by weight) | 2.9 | 3.90 | 1.21 | 3.2 |
| Example 20 | epichlorohydrin heptane/50°C | 7.4 mg (3.0% by weight) | 2.6 | 3.84 | 0.42 | 9.1 |
| Example 21 | epichlorohydrin heptane/50°C | 18.4 mg (7.4% by weight) | 2.4 | 3.79 | 0.58 | 6.5 |

(Example 22)

Urinary Phosphate Excretion Test of Normal Rats Using Surface Cross-Linked Polymer

(Synthesis of Sample to Be Tested and *in Vitro* Assay Thereof)

In 90.0 mL of ethanol, 4.50 g of the free-form cross-linked polyallylamine obtained in Example 7 was dispersed, and the resulting mixture was heated in an oil bath with stirring, to achieve an inner temperature of 50°C. In 45 mL of ethanol, 0.166 g of 2-hydroxyethyl acrylate (3.7% by weight with respect to the free-form cross-linked polyallylamine) was dissolved, and the resulting solution was added to the above mixture. After 0.5 hours of stirring, solids were recovered by filtration and washed with ethanol. This was followed by drying under reduced pressure at room temperature, and the solids were then dispersed in 50.0 mL of water. To the resulting mixture, 47.8 mL of 1 M hydrochloric acid was added with ice cooling and stirring. After stirring the resulting mixture at room temperature for 40 minutes, solids were recovered by filtration. The recovered solids were washed with water and then ethanol. The solids were dried under reduced pressure at room temperature to obtain 6.78 g of a cross-linked polyallylamine 2/3 hydrochloride (surface cross-linked product). Thereafter, 6.00 g of the obtained cross-linked polyallylamine 2/3 hydrochloride (surface cross-linked product) was freeze-crushed to obtain 5.99 g of a freeze-crushed product of a cross-linked polyallylamine 2/3 hydrochloride (surface cross-linked product). The obtained freeze-crushed product of a cross-linked polyallylamine 2/3 hydrochloride (surface cross-linked product) was assayed in the same manner as in Reference Example 6. The results are shown in Table 13. The assay results of the sevelamer hydrochloride prepared in Reference Example 6 are shown as Comparative Example 3.

[Table 13]

| Assay Results of Polymer for *in Vivo* Assay of Surface Cross-linked Product | | | | |
|---|---|---|---|---|
| | Degree of swelling | Phosphate adsorption capacity (mmol/g) | Bile acid adsorption capacity (mmol/g) | Phosphate selectivity |
| Example 22 | 3.1 | 3.97 | 1.47 | 2.7 |
| Comparative Example 3 (Reference Example 6) | 6.2 | 2.70 | 2.30 | 1.2 |

(*In Vivo* Assay)

SD rats (SPF, male, 6 weeks old, JAPAN SLC) were separately kept under a time-restricted feeding schedule of 8 hours/day with a feeding amount of 10 g/day. After about 1 week of habituation, the cross-linked polyallylamine 2/3 hydrochloride (surface cross-linked product) in Example 22 or sevelamer hydrochloride (Comparative Example 3 (Reference Example 6)) was mixed with a feed in an amount of 0.3% by weight or 1% by weight, and the rats were fed with the mixture for 3 days. Rats in the control group were fed with only the feed. During the 3 days of feeding, urine was collected for 24 hours/day and the daily urinary phosphate excretion amounts were measured to obtain the total sum of the amount during the 3 days. A decrease in the urinary phosphate excretion amount indicates the phosphate adsorption effect by the cross-linked polyallylamine 2/3 hydrochloride (surface cross-linked product) or sevelamer hydrochloride.

The results are shown in Fig. 3. The cross-linked polyallylamine 2/3 hydrochloride (surface cross-linked product) in Example 22 showed a dose-dependent and significant decrease in the phosphate excretion amount and the same level of the urinary phosphate excretion-reducing effect as sevelamer hydrochloride. Thus, it was shown that, while the cross-linked polyallylamine 2/3 hydrochloride (surface cross-linked product) in Example 22 has a lower degree of swelling than sevelamer hydrochloride, it has the same level of the phosphate adsorption effect as sevelamer hydrochloride.

(Reference Example 13)

Tablets of Renagel (registered trademark) which is a formulation containing as an effective component sevelamer hydrochloride (hereinafter referred to as "Renagel tablets") were pulverized using a grinder (A10, Junke&Kunkel IKA Labortechnic). The assay results of the pulverized product of Renagel tablets are shown in Table 14. The phosphate selectivity was low and the degree of swelling was 6.7.

[Table 14]

| Assay Results of Pulverized Product of Renagel Tablets | | | | |
|---|---|---|---|---|
| | Degree of swelling | Phosphate adsorption capacity (mmol/g) | Bile acid adsorption capacity (mmol/g) | Phosphate selectivity |
| Reference Example 13 | 6.7 | 3.36 | 2.51 | 1.3 |

(Example 23)

(Example of Synthesis by Reversed Phase Suspension Polymerization and *in Vitro* Assay)

In a 3 L three-necked flask, 17.9 g of sorbitan monolaurate and 1.26 kg of heptane were placed, and the atmosphere in the flask was replaced with nitrogen. The mixture was bubbled with nitrogen for 30 minutes with stirring. In a 200 mL three-necked flask, 12.9 g (4.00 mmol) of 2,2'-azobis[2-(2-imidazolin-2-yl)propane] dihydrochloride and 25 mL of water were placed, and the atmosphere in the flask was replaced with nitrogen with stirring. In a 5 L four-necked flask equipped with a mechanical stirrer and a thermometer, 155 g (1.00 mol) of allylammonium dihydrogen phosphate, 70.1 g (0.200 mol) of *N*,*N*'-diallyl-1,3-diaminopropane bis(dihydrogen phosphate) and 75 mL of water were placed, and the resulting mixture was heated at an inner temperature of 50°C to dissolve the compounds, followed by replacing the atmosphere in the flask to nitrogen. The aqueous suspension of the 2,2'-azobis[2-(2-imidazolin-2-yl)propane] dihydrochloride prepared in advance was added to the resulting solution, and the solution of sorbitan monolaurate in heptane prepared in advance was further added thereto. After stirring the resulting mixture at an inner temperature of 50 to 55°C for 20 hours, the mixture was cooled to room temperature. To the mixture, 500 mL of ethanol was added and the resulting mixture was filtered, followed by washing 3 times with 500 mL of ethanol, 7 times with 500 mL of water and twice with 500 mL of ethanol. The obtained solids were dried under reduced pressure at 50°C to obtain 218 g of a cross-linked polyallylamine phosphate. In a 5 L four-necked flask, the obtained polyallylamine phosphate and 2 L of water were placed, and 1.2 L of 20% aqueous sodium hydroxide solution was added to the resulting mixture with stirring by a mechanical stirrer. After 1 hour of stirring at room temperature, the mixture was filtered. The solids were washed 8 times with 1 L of water and 3 times with 250 mL of ethanol. The obtained solids were dried under reduced pressure at 55°C. A 10-g aliquot thereof was suspended in 200 mL of water and the suspension was stirred vigorously with a mechanical stirrer for 15 hours, followed by washing with water and then ethanol. The solids were dried under reduced pressure at 50°C to obtain a free-form cross-linked polyallylamine. The obtained free-form cross-linked polyallylamine was converted

to 20% acetate, 40% acetate and hydrochloride.

(20% Acetate (Example 23-1))

In 24 mL of water, 3.00 g of the free-form cross-linked polyallylamine was suspended, and 0.574 g of acetic acid dissolved in 6 mL of water was added to the suspension. After stirring the resulting mixture at room temperature for 30 minutes, the mixture was filtered, and the solids were washed with water and then ethanol. The solids were dried under reduced pressure at 40°C to obtain 3.61 g of 20% acetate of the cross-linked polyallylamine.

(40% Acetate (Example 23-2))

In 24 mL of water, 3.00 g of the free-form cross-linked polyallylamine was suspended, and 1.15 g of acetic acid dissolved in 6 mL of water was added to the suspension. After stirring the resulting mixture at room temperature for 30 minutes, the mixture was filtered, and the solids were washed with water and then ethanol. The solids were dried under reduced pressure at 40°C to obtain 4.18 g of 20% acetate of the cross-linked polyallylamine.

(Hydrochloride (Example 23-3))

In 25 mL of water, 0.200 g of the free-form cross-linked polyallylamine was suspended, and 1 mL of concentrated hydrochloric acid was added to the suspension. After stirring the resulting mixture at room temperature for 30 minutes, the mixture was filtered, and the solids were washed with water and then ethanol. The solids were dried under reduced pressure at 50°C to obtain 0.293 g of a cross-linked polyallylamine hydrochloride.

The three obtained samples of the acid addition salts of the cross-linked polyallylamine were assayed in the same manner as in Reference Example 6. The results are shown in Table 15. The assay results of the pulverized product of Renagel tablets prepared in Reference Example 13 are shown as Comparative Example 5. All the salts showed a lower degree of swelling, a higher phosphate adsorption capacity and a higher phosphate selectivity than the pulverized product of Renagel tablets.

[Table 15]

| Assay Results of Cross-linked Polyallylamine Acetate and Hydrochloride Obtained by Reversed Phase Suspension Polymerization | | | | | |
|---|---|---|---|---|---|
| | Acid added and salt formation rate | Degree of swelling | Phosphate adsorption capacity (mmol/g) | Bile acid adsorption capacity (mmol/g) | Phosphate selectivity |
| Comparative Example 5 (Reference Example 13) | - | 6.7 | 3.36 | 2.51 | 1.3 |
| Example 23-1 | acetic acid (20%) | 3.1 | 3.39 | 0.74 | 4.6 |
| Example 23-2 | acetic acid (40%) | 3.1 | 4.03 | 0.50 | 8.1 |
| Example 23-3 | hydrochloric acid (100%) | 2.7 | 4.11 | 1.05 | 3.9 |

(*In Vivo* Assay)

SD rats (SPF, male, 6 weeks old, JAPAN SLC) were separately kept under a time-restricted feeding schedule of 8 hours/day with a feeding amount of 10 g/day. After about 1 week of habituation, the pulverized product of Renagel tablets (Reference Example 13) or the cross-linked polyallylamine acetate in Example 23-1 or Example 23-2 was mixed with a feed in an amount shown in Table 16, and the rats were fed with the mixture for 3 days. Rats in the control group were fed with only the feed. Using sevelamer hydrochloride contained in the pulverized product of Renagel tablets as

the standard, the amount of the polymer was set to attain the same weight as the free form. During the 3 days of feeding, urine was collected for 24 hours/day and the daily urinary phosphate excretion amounts were measured to obtain the total sum of the amount during the 3 days. A decrease in the urinary phosphate excretion amount indicates the phosphate adsorption effect by the polymer.

The results are shown in Fig. 4. The polymers in Example 23-1 and Example 23-2 showed significant decreases in the phosphate excretion amount, and showed the same level of the urinary phosphate excretion-reducing effect as the pulverized product of Renagel tablets (Reference Example 13). Thus, the polymers in Example 23-1 and Example 23-2 were shown to have the same level of the phosphate adsorption effect as the pulverized product of Renagel tablets, while having a lower degree of swelling than that of the pulverized product of Renagel tablets.

[Table 16]

| Dosage of Samples in Example 23 | |
|---|---|
| | Dosage (mg) |
| Control | 0 |
| Comparative Example 5 (Reference Example 13) | 120 |
| Example 23-1 | 98 |
| Example 23-2 | 114 |

(Example 24)

(Example of Synthesis by Precipitation Polymerization and *in Vitro* Assay)

To a 100 mL four-necked flask equipped with a mechanical stirrer, 9.31 (60 mmol) of allylammonium dihydrogen phosphate, 4.20 g (12 mmol) of *N*,*N*'-diallyl-1,3-diaminopropane bis(dihydrogen phosphate), 12 mL of water and 12 mL of ethanol were placed, and the atmosphere in the system was replaced 3 times with argon, followed by addition thereto 0.814 g (3 mmol) of 2,2'-azobis(2-amidinopropane) dihydrochloride and heating the resulting mixture at a bath temperature of 60 to 65°C with stirring for 20 hours. The obtained solids were recovered by filtration and washed with water and then ethanol. The solids were dried under reduced pressure at room temperature to obtain 10.3 g of powder of a cross-linked polyallylamine phosphate. In 54 mL of water, 9.00 g of the obtained cross-linked polyallylamine phosphate was dispersed, and 36 mL of 20% aqueous sodium hydroxide solution was added thereto with stirring with a magnetic stirrer. The resulting mixture was stirred at room temperature for 1 hour, and solids were recovered by filtration, followed by washing thereof with water until the filtrate becomes neutral. The solids were washed with ethanol and dried under reduced pressure at 50°C to obtain 3.5 g of a free-form cross-linked polyallylamine.

While stirring 2.26 g of the free-form cross-linked polyallylamine by a magnetic stirrer, 24 mL of 1 M hydrochloric acid was added thereto. After 1 hour of stirring at room temperature, solids were recovered by filtration. The recovered solids were washed with water and then ethanol sufficiently. The solids were dried under reduced pressure at room temperature to obtain 3.14 g of a cross-linked polyallylamine 2/3 hydrochloride. The obtained cross-linked polyallylamine 2/3 hydrochloride was assayed in the same manner as in Reference Example 6. The results are shown in Table 17. The assay results of the pulverized product of Renagel tablets prepared in Reference Example 13 are shown as Comparative Example 5. The polymer obtained by precipitation polymerization showed a lower degree of swelling, a higher phosphate adsorption capacity and a higher phosphate selectivity.

[Table 17]

| Assay Results of Polymer Obtained by Precipitation Polymerization | | | | |
|---|---|---|---|---|
| | Degree of swelling | Phosphate adsorption capacity (mmol/g) | Bile acid adsorption capacity (mmol/g) | Phosphate selectivity |
| Comparative Example 5 (Reference Example 13) | 6.7 | 3.36 | 2.51 | 1.3 |

(continued)

| Assay Results of Polymer Obtained by Precipitation Polymerization | | | | |
|---|---|---|---|---|
| | Degree of swelling | Phosphate adsorption capacity (mmol/g) | Bile acid adsorption capacity (mmol/g) | Phosphate selectivity |
| Example 24 | 4.0 | 4.50 | 0.85 | 5.3 |

(*In Vivo* Assay)

SD rats (SPF, male, 6 weeks old, JAPAN SLC) were separately kept under a time-restricted feeding schedule of 8 hours/day with a feeding amount of 10 g/day. After about 1 week of habituation, sevelamer hydrochloride contained in the pulverized product of Renagel tablets (Reference Example 13) or the polymer in Example 24 was mixed with a feed in an amount of 1% by weight, and the rats were fed with the mixture for 3 days. Rats in the control group were fed with only the feed. During the 3 days of feeding, urine was collected for 24 hours/day and the daily urinary phosphate excretion amounts were measured to obtain the total sum of the amount during the 3 days. A decrease in the urinary phosphate excretion amount indicates the phosphate adsorption effect by the polymer.
The results are shown in Fig. 5. The polymer in Example 24 showed a significant decrease in the phosphate excretion amount, and showed the same level of the urinary phosphate excretion-reducing effect as the pulverized product of Renagel tablets (Reference Example 13). Thus, the polymer in Example 24 was shown to have the same level of the phosphate adsorption effect as the pulverized product of Renagel tablets, while having a lower degree of swelling than that of the pulverized product of Renagel tablets.

(Example 25)

Into a polypropylene test tube, 500 mg of the allylamine-type polymer synthesized by the method in Example 15 or the pulverized product of Renagel tablets prepared in Reference Example 13 was weighed, and a 50 mg/mL suspension was prepared with the second fluid of the disintegration test described in Japanese Pharmacopoeia 14th revised edition (0.05 mol/L potassium dihydrogen phosphate, 0.0236 mol/L sodium hydroxide, pH about 6.8). After the preparation, the suspension was rotated on a wave rotor (Thermonics, WR-40) to avoid precipitation of the test substance. The colon was isolated from a Sprague Dawley (SD) rat (SPF, male, 7 weeks old, JAPAN SLC) under anesthesia with ether and washed with cold physiological saline, followed by excision of 2 pieces with a length of 5 cm. The intestinal tract was reversed using a sonde for mice, which sonde had been weighed in advance (pre), and the both ends of the intestinal tract were knotted by surgical suture to fix the intestinal tract to the sonde, followed by measuring the weight of the intestinal tract + the sonde (pre). In each of the polypropylene tubes containing the test substance solutions stirred in advance with the wave rotor, the intestinal tract + the sonde was placed such that it does not move, and the tubes were rotated on the wave rotor for 5 minutes. Five minutes later, the intestinal tract + the sound were recovered from each polypropylene tube, and the weight of the intestinal tract + the sonde (post) and the weight of the sonde (post) were measured. The amount of each test substance solution adhered to the intestinal tract was calculated according to the formula below.

$$\{[\text{the weight of the intestinal tract + the sonde (post)}] - [\text{the weight of the intestinal tract + the sonde (pre)}]\} - \{[\text{the sonde (post)}] - [\text{the sonde (pre)}]\}$$

The results are shown in Fig. 6. The weights of the polymers synthesized by the method in Example 15 adhered to the colon were significantly lower compared to those of the pulverized products of Renagel tablets (Comparative Example 6).

(Example 26)

Intestinal tract-adherence tests were carried out on the polymers with and without surface cross-linking, which were obtained by the reversed phase suspension polymerization. The polymers to be assayed were synthesized using a free-form cross-linked polyallylamine synthesized according to the method shown in Example 23.

(Synthesis of Polymer without Surface Cross-linking (Example 26-1))

In 80 mL of water, 4.00 g of a free-form cross-linked polyallylamine was suspended, and 80 mL of 1 M hydrochloric acid was added thereto with stirring. After 30 minutes of stirring at room temperature, solids were recovered by filtration. The solids were washed with water and then ethanol. The solids were then dried under reduced pressure at 50°C to obtain 6.45 g of a cross-linked polyallylamine hydrochloride.

(Synthesis of Polymer with Surface Cross-linking (Example 26-2))

In 80 mL of ethanol, 4.00 g of a free-form cross-linked polyallylamine was dispersed, and the resulting mixture was stirred at 30°C. To the mixture, 0.370 g of 2-hydroxyethyl acrylate dissolved in 20 mL of ethanol was added, and the resulting mixture was stirred for 30 minutes. Solids were recovered by filtration and washed with ethanol, followed by drying under reduced pressure at room temperature. The obtained polymer was dispersed in 80 mL of water, and 80 mL of 1 M hydrochloric acid was added thereto with ice cooling and stirring. After 30 minutes of stirring at room temperature, solids were recovered by filtration. The recovered solids were washed with water and then ethanol. This was followed by drying under reduced pressure at room temperature to obtain 6.70 g of a cross-linked polyallylamine hydrochloride (surface cross-linked product).

(Intestinal Tract-adherence Test)

Intestinal tract-adherence tests were carried out by the method shown in Example 25. The results are shown in Fig. 7. The cross-linked polyallylamine hydrochloride (surface cross-linked product) obtained by the reversed phase suspension polymerization showed significantly lower values of the weight of adherence to the colon compared to the pulverized products of Renagel tablets (Comparative Example 6) irrespective of whether or not the surface cross-linking was carried out.

INDUSTRIAL APPLICABILITY

Since the cross-linked polyallylamine or the acid addition salt thereof of the present invention has a high phosphate adsorption capacity and phosphate selectivity, and a remarkably lower degree of swelling than the prior art, it is appropriate as a pharmaceutical agent having less side effects such as constipation, abdominal pain and abdominal distension, especially as a therapeutic or prophylactic agent for hyperphosphatemia.

## Claims

1. A cross-linked polyallylamine or an acid addition salt thereof, which is obtained by copolymerization of allylammonium dihydrogen phosphate with an acid addition salt of *N*,*N*'-diallyl-1,3-diaminopropane in an amount of 5 to 25 mol% with respect to the amount of said allylammonium dihydrogen phosphate, said cross-linked polyallylamine or an acid addition salt thereof having:

   a phosphate adsorption capacity of 2.7 to 5.0 mmol/g; and
   a degree of swelling of 2.0 to 5.0.

2. The cross-linked polyallylamine or an acid addition salt thereof according to claim 1, whose surface was cross-linked by reacting a compound having 2 or more amino group-reactive functional groups with an amino group.

3. The cross-linked polyallylamine or an acid addition salt thereof according to claim 2, wherein said compound having 2 or more amino group-reactive functional groups is an acrylic acid ester, methacrylic acid ester, epihalohydrin, dihalogenated hydrocarbon, diepoxide or dibasic acid chloride.

4. The cross-linked polyallylamine or an acid addition salt thereof according to claim 2, wherein said compound having 2 or more amino group-reactive functional groups is an acrylic acid ester.

5. The cross-linked polyallylamine or an acid addition salt thereof according to any one of claims 1 to 4, wherein the acid addition salt of said *N*,*N*'-diallyl-1,3-diaminopropane is *N*,*N*'-diallyl-1,3-diaminopropane bis(dihydrogen phosphate).

**6.** A pharmaceutical composition comprising as an effective ingredient the cross-linked polyallylamine or an acid addition salt thereof according to any one of claims 1 to 5.

**7.** A therapeutic or prophylactic agent for hyperphosphatemia, comprising as an effective ingredient the cross-linked polyallylamine or an acid addition salt thereof according to any one of claims 1 to 5.

**8.** A therapeutic or prophylactic method for hyperphosphatemia, comprising administration of an effective amount of the cross-linked polyallylamine or an acid addition salt thereof according to any one of claims 1 to 5 to a patient for whom therapy or prophylaxis of hyperphosphatemia is desired.

**9.** Use of the cross-linked polyallylamine or an acid addition salt thereof according to any one of claims 1 to 5, for production of a therapeutic or prophylactic agent for hyperphosphatemia.

**10.** A compound for therapy or prophylaxis of hyperphosphatemia, which is the cross-linked polyallylamine or an acid addition salt thereof according to any one of claims 1 to 5.

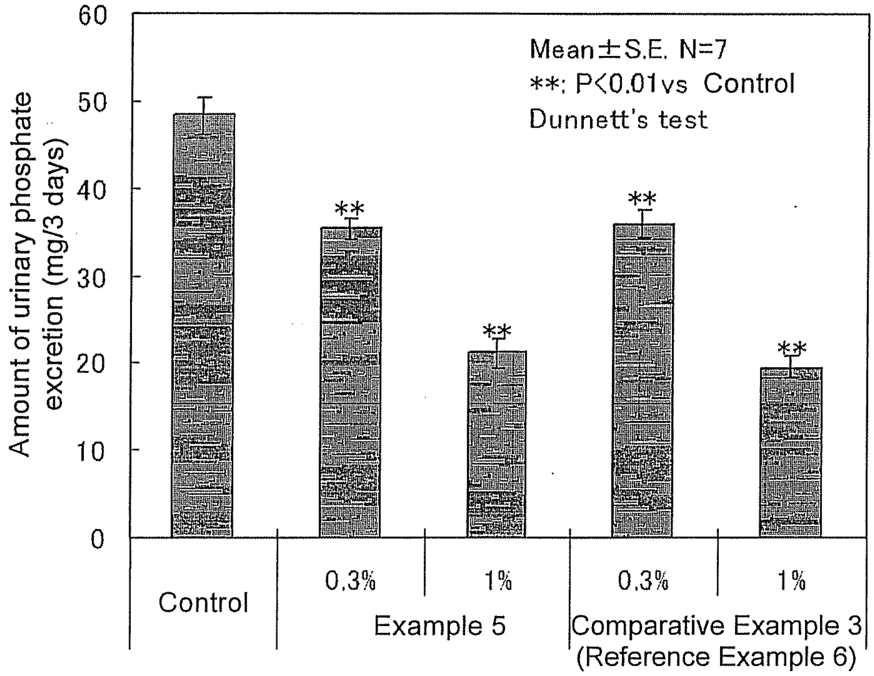
60
50
40
30
20
10
0
Amount of urinary phosphate excretion (mg/3 days)
Mean±S.E. N=7
**: P<0.01vs Control
Dunnett's test
**
**
**
**
Control
0.3%
1%
0.3%
1%
Example 5
Comparative Example 3 (Reference Example 6)

**Fig.1**

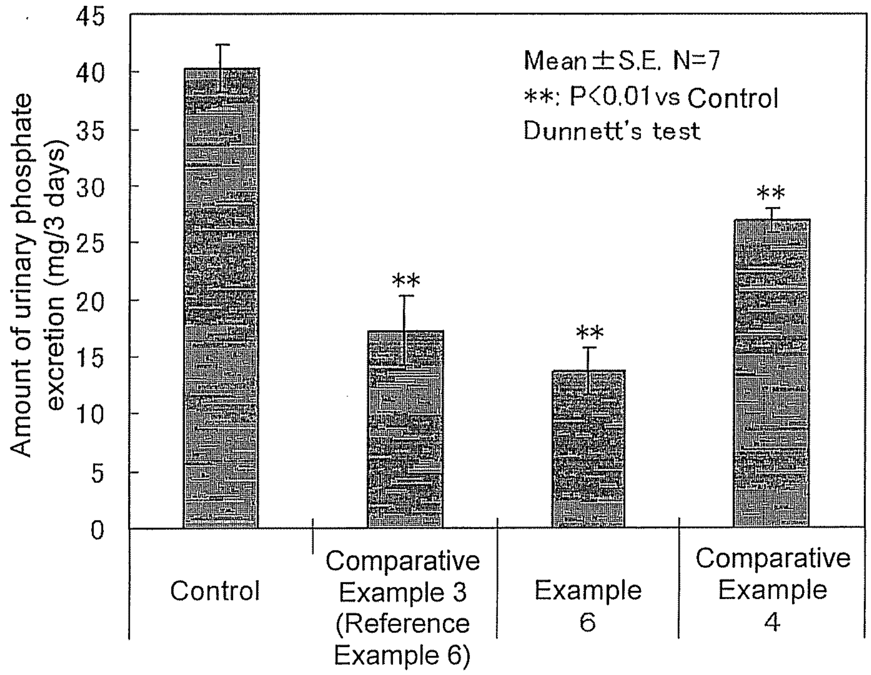
45
40
35
30
25
20
15
10
5
0
Amount of urinary phosphate excretion (mg/3 days)
Mean±S.E. N=7
**: P<0.01vs Control
Dunnett's test
**
**
**
Control
Comparative Example 3 (Reference Example 6)
Example 6
Comparative Example 4

**Fig.2**

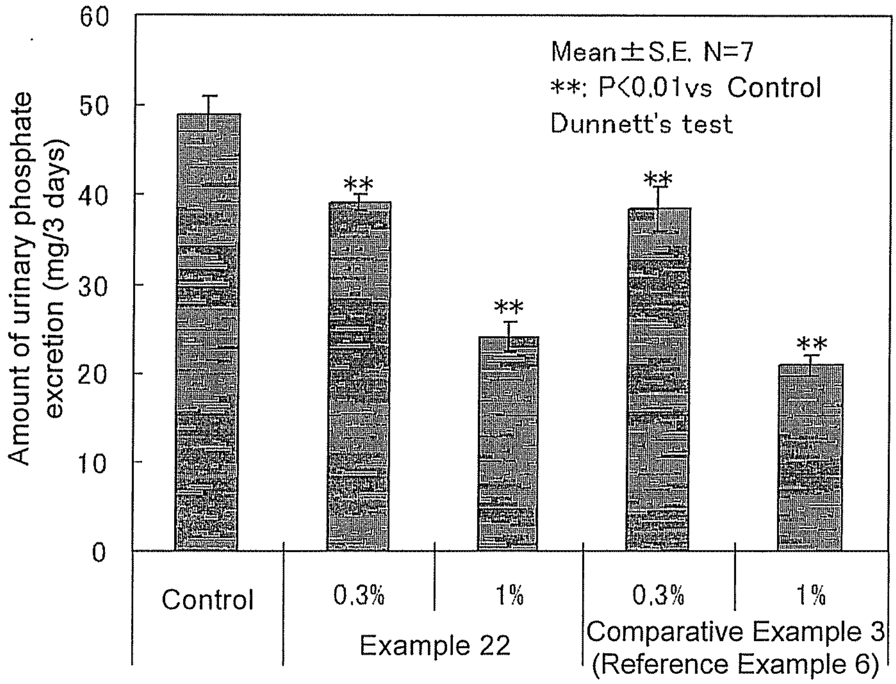
Amount of urinary phosphate excretion (mg/3 days)
Mean±S.E. N=7
**: P<0.01vs Control
Dunnett's test
Control
0.3%
1%
Example 22
0.3%
1%
Comparative Example 3 (Reference Example 6)

**Fig.3**

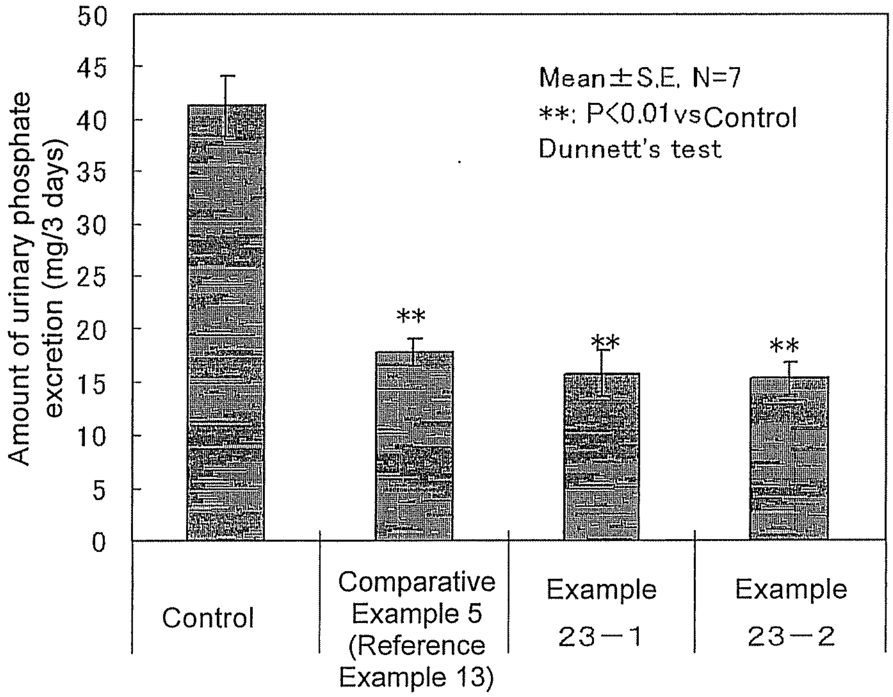
Amount of urinary phosphate excretion (mg/3 days)
Mean±S.E. N=7
**: P<0.01vsControl
Dunnett's test
Control
Comparative Example 5 (Reference Example 13)
Example 23−1
Example 23−2

**Fig.4**

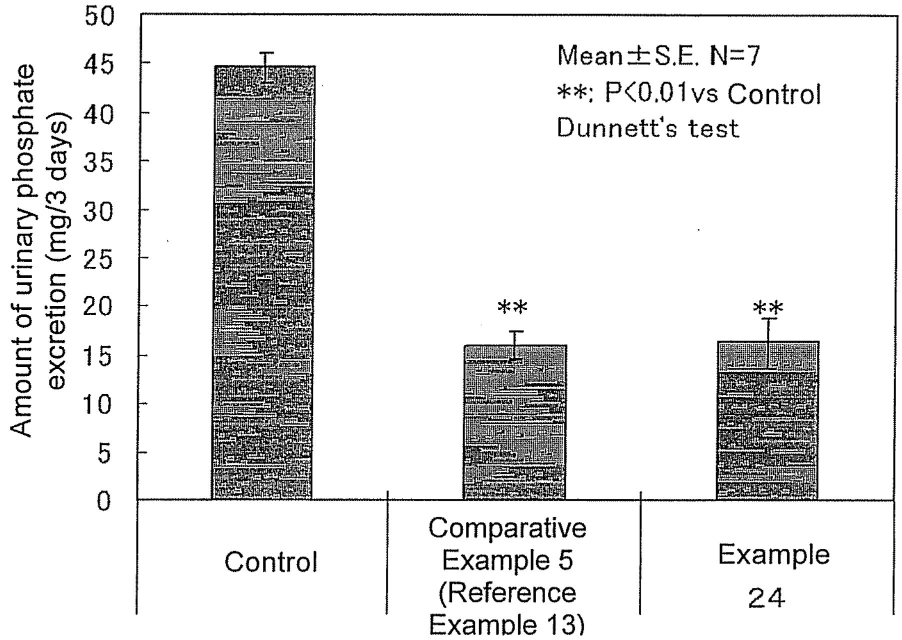
Amount of urinary phosphate excretion (mg/3 days)
50
45
40
35
30
25
20
15
10
5
0
Mean±S.E. N=7
**; P<0.01vs Control
Dunnett's test
**
**
Control
Comparative Example 5 (Reference Example 13)
Example 24

Fig.5

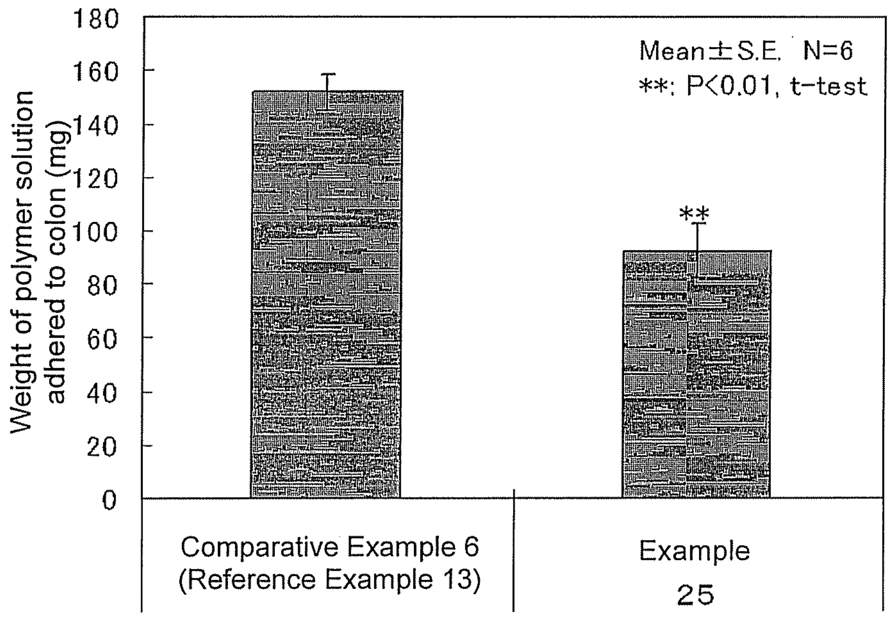
Weight of polymer solution adhered to colon (mg)
180
160
140
120
100
80
60
40
20
0
Mean±S.E. N=6
**; P<0.01, t-test
**
Comparative Example 6 (Reference Example 13)
Example 25

Fig.6

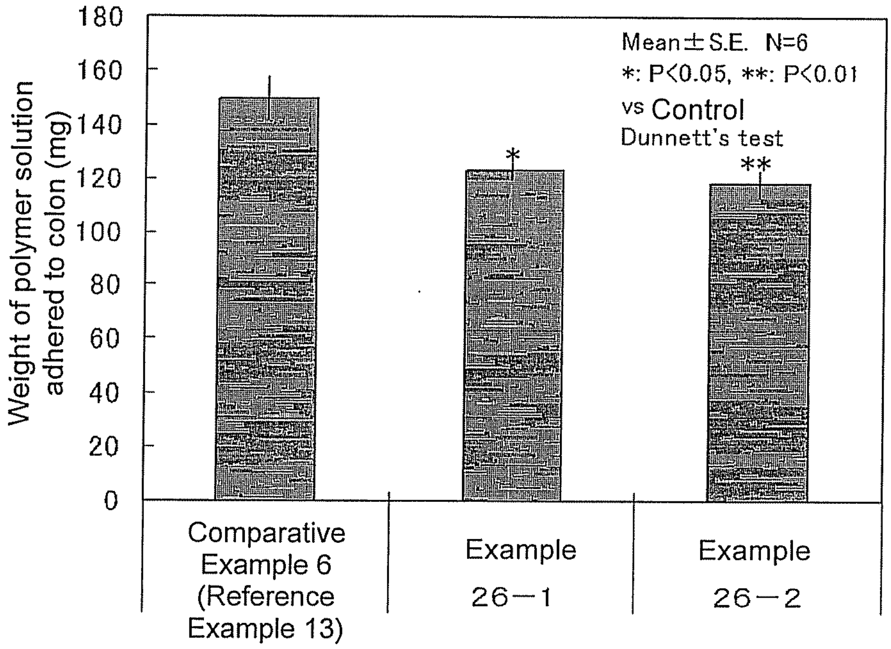
Weight of polymer solution adhered to colon (mg)
180
160
140
120
100
80
60
40
20
0
Mean±S.E. N=6
*: P<0.05, **: P<0.01
vs Control
Dunnett's test
*
**
Comparative Example 6 (Reference Example 13)
Example 26−1
Example 26−2

**Fig.7**

**INTERNATIONAL SEARCH REPORT**

International application No.
PCT/JP2008/062505

A. CLASSIFICATION OF SUBJECT MATTER
*C08F230/02*(2006.01)i, *A61K31/785*(2006.01)i, *A61P3/12*(2006.01)i

According to International Patent Classification (IPC) or to both national classification and IPC

B. FIELDS SEARCHED

Minimum documentation searched (classification system followed by classification symbols)
C08F230/02, A61K31/785, A61P3/12

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 58-201811 A (Nitto Boseki Co., Ltd.), 24 November, 1983 (24.11.83), Full text & AU 1292983 A & CA 1233945 A1 & DE 3363895 D1 & EP 095233 A2 & KR 890002931 B1 & US 4504640 A | 1-7,9-10 |
| A | JP 10-330427 A (Nitto Boseki Co., Ltd.), 15 December, 1998 (15.12.98), Full text (Family: none) | 1-7,9-10 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>22 September, 2008 (22.09.08) | Date of mailing of the international search report<br>07 October, 2008 (07.10.08) |
| Name and mailing address of the ISA/<br>Japanese Patent Office<br>Facsimile No. | Authorized officer<br><br>Telephone No. |

Form PCT/ISA/210 (second sheet) (April 2007)

INTERNATIONAL SEARCH REPORT

International application No.
PCT/JP2008/062505

C (Continuation). DOCUMENTS CONSIDERED TO BE RELEVANT

| Category* | Citation of document, with indication, where appropriate, of the relevant passages | Relevant to claim No. |
|---|---|---|
| A | WO 00/52070 A1 (Nitto Boseki Co., Ltd.), 08 September, 2000 (08.09.00), Full text & CA 2330663 A1 & DE 69931369 D1 & EP 1083191 A1 & JP 3937287 B2 & US 6579933 B1 | 1-7,9-10 |
| A | JP 2002-542345 A (Abbott Laboratories), 10 December, 2002 (10.12.02), Full text & AT 366264 T & CA 2362410 A1 & DE 60035414 D1 & DK 1175451 T3 & EP 1175451 A1 & EP 1881013 A1 & ES 2288852 T3 | 1-7,9-10 |

Form PCT/ISA/210 (continuation of second sheet) (April 2007)

**INTERNATIONAL SEARCH REPORT**

International application No.
PCT/JP2008/062505

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 8
because they relate to subject matter not required to be searched by this Authority, namely:
The invention of claim 8 is relevant to a method for treatment of a human body by therapy and thus relates to a subject matter which this International Searching Authority is not required, under the provisions of PCT Article 17(2)(a)(i) and PCT Rule 39.1(iv), to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3113283 B
- JP 10330427 A
- JP 2014364 B
- US 20050276781 A1
- JP 11302391 A
- JP 3113283 A


### Non-patent literature cited in the description


- Drug Interview Form: Renagel. 2005, 21
- *Review Report, Notification No. 3850 of National Institute of Health Sciences,* 28 November 2002
- **Hiroyoshi INOUE.** Phosphate excretion enhancers/absorption inhibitors in the future. *Kidney and Dialysis,* 2003, vol. 55, 941-944